(19) Europäisches Patentamt
European Patent Office
Office européen des brevets

(11) Numéro de publication: **0 217 688**
**B1**

(12) **FASCICULE DE BREVET EUROPEEN**

(45) Date de publication du fascicule du brevet: **26.09.90**

(21) Numéro de dépôt: **86401738.9**

(22) Date de dépôt: **04.08.86**

(51) Int. Cl.$^5$: **C 07 K 5/06,** C 07 K 5/08, A 61 K 37/02

# (54) Nouveaux dérivés peptidiques à structure lactonique ou cycloamidique, leur procédé de préparation et les compositions pharmaceutiques qui les contiennent.

(30) Priorité: **05.08.85 FR 8511936**

(43) Date de publication de la demande:
**08.04.87 Bulletin 87/15**

(45) Mention de la délivrance du brevet:
**26.09.90 Bulletin 90/39**

(84) Etats contractants désignés:
**AT BE CH DE FR GB IT LI LU NL SE**

(56) Documents cités:
**DE-A-3 332 633**
**GB-A-2 130 221**
**US-A-4 293 481**

(73) Titulaire: **ADIR ET COMPAGNIE**
**1 rue Carle Hébert**
**F-92415 Courbevoie Cédex (FR)**

(72) Inventeur: **Vincent, Michel**
**8, allée du Prunier Hardy**
**F-92220 Bagneux (FR)**
Inventeur: **Remond, Georges**
**9 avenue des Etats-Unis**
**F-78000 Versailles (FR)**
Inventeur: **Lepagnol, Jean**
**5 rue M. de Vlaminck**
**F-78400 Chatou (FR)**

**EP 0 217 688 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne de nouveaux dérivés peptidiques à structure lactonique ou cycloamidique, leur procédé de préparation et les compositions pharmaceutiques qui les contiennent.

On connait quelques tripeptides endogènes à structure cyclo-amidique, en particulier la TRH (pyroglutamyl-histidyl-prolinamide) qui possède des effets endocriniens (stimulation de la sécrétion de TSH et de prolactine) et des effets comportementaux au niveau du système nerveux central, passant par une stimulation de libération d'acétyl-choline au niveau de l'hippocampe (stimulations motrice, de l'éveil ou de la mémorisation).

Cependant, la TRH est rapidement inactivée métaboliquement dans l'organisme en 2 à 5 minutes.

D'autres tripeptides ont été décrits (brevets FR.2.187.155 et 2.287.916, 2.266.515, 2.345.448) dans lesquels le reste pyroglutamyle est remplacé par un autre reste d'acide hétérocyclique carboxylique, et notamment les acides oxo-2 tétrahydrofuranne carboxylique-5, tétrahydro-1,2,5,6 dioxo-2,6 pyrimidine carboxylique-4 ou oxo-2 pipéridinyle carboxylique-6. Ces composés possédent une activité anti-convulsivante et anti-dépressive.

Les composés de la présente invention, dans lesquels le reste prolinamide est remplacé par une structure bicyclique saturée, présentent les propriétés très intéressantes de stimuler la synthèse d'AMP cyclique au niveau du tissu cérébral, donc d'augmenter les capacités métaboliques du cerveau, et d'améliorer les capacités de neurotransmission centrale tant cholinergique que catécholaminergique tout en manifestant une résistance vis à vis des enzymes tissulaires ou plasmatiques.

L'invention concerne plus particulièrement de nouveaux dérivés à structure lactonique ou cycloamidique, réspondant à la formule générale:

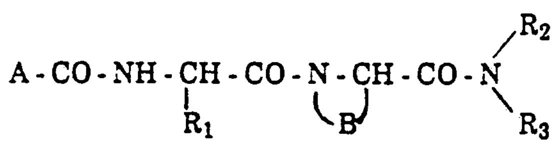

(I)

dans laquelle:

A représente un groupement oxo-2 tétrahydrofuryle-5, oxo-2 pyrrolidinyle-5 ou oxo-2 pipéridinyle-6 éventuellement substitué au niveau de l'atome d'azote par un groupement alkyle de 1 à 4 atomes de carbone, en chaîne droite ou ramifiée, ou un groupement dioxo-2,6 tétrahydro-1,2,3,6pyrimidinyle-4,

B représente, avec l'atome de carbon et l'atome d'azote auxquels il est attaché une structure polycyclique saturée choisie parmi le groupe constitué des perhydroindole, perhydroisoindole, perhydroquinoléïne, perhydroisoquinoléïne, cyclopenta [b] pyrrole, et aza-2 bicyclo[2,2,2]octane,

$R_1$ représente un atome d'hydrogène, un radical alkyle renfermant de 1 à 4 atomes de carbone en chaîne droite ou ramifiée, ou un groupe (imidazolyl-4) méthyle éventuellement substitué au niveau d'un des atomes d'azote par un radical alkyle de 1 à 4 atomes de carbone en chaîne droite ou ramifiée ou par un radical benzyloxycarbonyle, dinitro-2,4, phényle fluorénométhyloxycarbonyle, tosyle ou benzyle,

$R_2$ et $R_3$, identiques ou différents, représentent un atome d'hydrogène, un radical alkyle renfermant de 1 à 4 atomes de carbone ou forment ensemble, avec l'atome d'azote auquel ils sont attachés, un radical pyrrolidinyle-1, pipéridinyle-1, morpholinyle-4 ou alkyl (de 1 à 4 atomes de carbone)-4 pipérazinyle-1,

leurs énantiomères et diastéréoisomères,

ainsi que, lorsque $R_1$ représente un groupe(imidazolyl-4) méthyle éventuellement substitué au niveau d'un des atomes d'azote par un radical alkyle de 1 à 4 atomes de carbone en chaîne droite ou ramifiée, ou lorsque $R_2$ et $R_3$ représentent un radical N-alkyle pipérazinyle-1, leurs sels d'addition à un acide pharmaceutiquement acceptable.

Parmi les acides pharmaceutiquement acceptables, on peut citer à titre non limitatif, les acides chlorhydrique, sulfurique, tartrique, maléïque, fumarique, oxalique, méthanesulfonique camphorique etc.

L'invention s'étend aussi au procédé de préparation des dérivés de formule I, caractérisé en ce que l'on protège la fonction aminée d'un amino acide de formule II

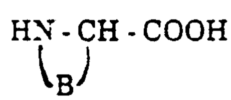

(II)

dans laquelle B avec les atomes d'azote et de carbone auxquels il est attaché a la même signification que dan la formule I, par un radical tertiobutoxycarbonyle (tBoc) sous l'action du carbonate de ditertiobutyle, en un dérivé de formule III.

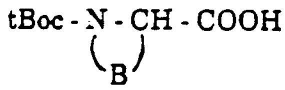

(III)

dans laquelle B a la même signification que dans la formule I,
qui est ensuite transformé, à une température comprise entre 0 et −15°C, par action successive du chloroformiate d'éthyle et d'une amine de formule IV.

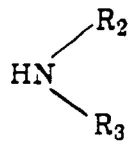

(IV)

dans laquelle $R_2$ et $R_3$ ont les mêmes significations que dans la formule I,
en un amide de formule V:

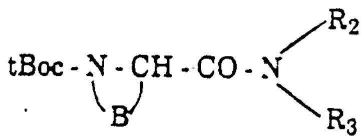

(V)

dans laquelle B, $R_2$ et $R_3$ ont les mêmes significations que dans la formule I,
que l'on déprotège, par action de l'acide chlorhydrique gazeux dans l'acétate d'éthyle ou de l'acide trifluoroacétique, en un dérivé de formule VI:

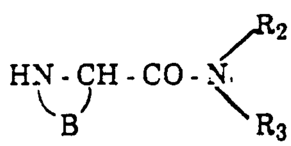

(VI)

dans laquelle B, $R_2$, $R_3$ ont les mêmes significations que dans la formule I, qui est couplé avec un amino acide protégé de formule VII,

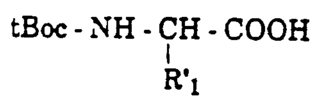

(VII)

dans laquelle $R'_1$ représente un atome d'hydrogène, un radical alkyle de 1 à 4 atomes de carbone en chaîne droite ou ramifiée, un radical (N-alkyl (de $C_1$ à $C_4$) imidazolyl)-4 méthyle, ou un radical imidazolyl-4 méthyle préalablement protégé qu niveau d'un des atomes d'azote par un groupement tel qu'un radical dinitro-2,4 phényle, benzyloxycarbonyle, fluorénométhyloxycarbonyle, tosyle, ou benzyle, en un dérivé de formule VIII,

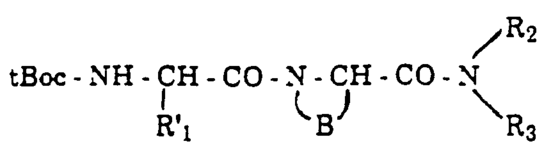

(VIII)

dans laquelle $R'_1$, $R_2$, $R_3$ et B ont les mêmes significations définies précédemment,
que l'on déprotège comme précédemment en un dérivé de formule IX,

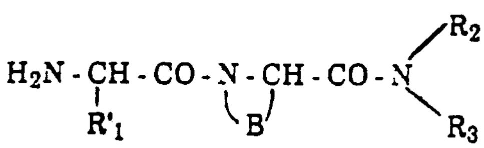

(IX)

dans laquelle $R'_1$, $R_2$, $R_3$ et B ont les mêmes significations que précédemment, qui est condensé avec un dérivé de formule X,

A—COOH (X)

dans laquelle A a les mêmes significations que dans la formule I,
*soit en un dérivé de formule I',

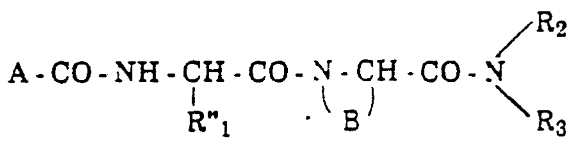

(I')

dans laquelle A, B, $R_2$, $R_3$ ont les mêmes significations que précédemment et $R''_1$ représente, un atome d'hydrogène, un radical alkyle de 1 à 4 atomes de carbone en chaîne droite ou ramifiée ou un radical (N-alkyle (de 1 à 4 atomes de carbone) imidazolyl-4) méthyle,
qui est, si l'on désire, directement transformé en son sel d'addition à un acide pharmaceutiquement acceptable lorsque R''1 représente un radical (N-alkyl imidazolyl) méthyle ou lorsque $R_2$ et $R_3$ représentent, avec l'atome d'azote auxquels ils sont attachés, un radical pipérazinyl-1 substitué par une chaîne alkyle de 1 à 4 atomes de carbone,
ou séparé au préalable en ses énantiomères ou diastéréoisomères, puis transformé si l'on désire, lorsque $R''_1$, $R_2$ et $R_3$ ont les mêmes significations que ci-dessus définies en son sel d'addition à un acide pharmaceutiquement acceptable,
*soit en un dérivé de formule I'':

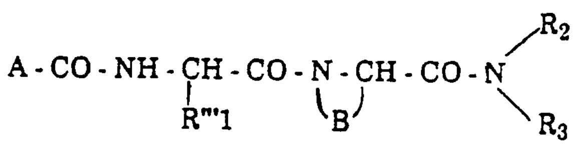

(I'')

dans laquelle A, B, $R_2$ et $R_3$ ont les mêmes significations que précédemment et $R'''_1$ représente un groupement imidazolyl-4 méthyle protégé au niveau d'un des atomes d'azote par un groupement, benzyloxycarbonyle, dinitro-2,4-phényle, fluorénométhyloxycarbonyle, tosyle, ou benzyle,
que l'on déprotège en un dérivé de formule I''',

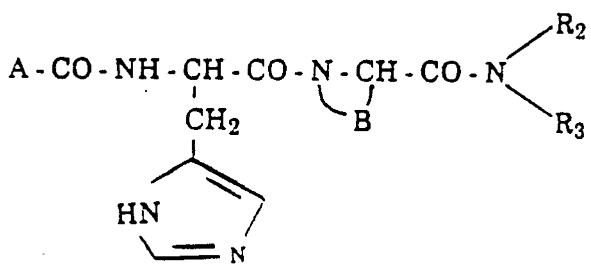

(I''')

dans lequel A, B, $R_2$ et $R_3$ gardent les significations ci-dessus définies,
qui est si l'on désire transformé en son sel d'addition à un acide pharmaceutiquement acceptable,
ou séparé en ses isomères puis si nécessaire salifié par un acide pharmaceutiquement acceptable.

L'ensembler des composés de formule I', I'' et I''' représente les composés de formule I.

Les composés de formule I possèdent des propriétés pharmacologiques très intéressantes chez l'animal, ils augmentent le contenu intracérébral d'AMPc et inhibent la narcose induite par le pentobarbital. Cette inhibition de la narcose traduit, en particulier, une activité au niveau de l'uptake de choline.

Les composés de formule I exercent en outre des effets stimulants vis à vis des voies de neurotransmission catécholaminergique puisqu'ils potentialisent la toxicité de la yohimbine chez la souris, le pic hypertensif à la noradrénaline chez le rat amyélé et puisqu'ils augmentent la locomotion spontanée et l'activité exploratoire chez la souris.

Or la baisse du taux d'AMPc avec l'âge et la moindre efficience de la neurotransmission cholinergique et catécholaminergique sont associées chez l'homme aux désordres mnésiques du vieillissement et aux démences séniles.

Les composés de l'invention sont donc utiles en thérapeutique dans le traitement des désordres du système nerveux central (par exemple, désordres de conscience, de langage, coma, autisme, syndrome hyperkinétique, schizophrénie, dépression, maladie de Parkinson . . .) et dans le traitement des désordres liés au vieillissement normal ou pathologique (démence sénile, maladie de Alzheimer) puisqu'ils en corrigent les désordres métaboliques en stimulant la neurotransmission et en restaurant par exemple le taux d'AMPc.

La présente invention a également pour objet les compositions pharmaceutiques renfermant comme principe actif au moins un composé de formule générale I ou un de ses sels d'addition à un acide pharmaceutiquement acceptable, seul ou en combinaison avec un ou plusieurs excipients ou véhicules, inertes, non-toxiques.

Parmi les compositions pharmaceutiques selon l'invention, on peut citer celles qui conviennent pour l'administration parentérale, orale, ou rectale, notamment les préparations injectables ou buvables, les comprimés, gélules, capsules, dragées, sachets, suppositoires . . .

La posologie varie selon l'âge et le poids du patient, la voie d'administration, la nature de l'affection et peut s'échelonner entre 1 à 200 mg par prise, pouvant être répétée 2 à 5 fois par jour.

Les exemples suivant illustrent l'invention et ne la limitent en aucune façon.

Les produits de départ sont connus de la littérature ou peuvent être préparés de façon similaire.

Les points de fusion indiqués sont mesurés selon la technique micro-Kofler. Les spectres de résonance magnétique nucléaire du proton ont été enregistrés à 60 MHz et en utilisant le $D_2O$ comme solvant et le TMS comme référence interne. Lorsque le solvant n'est pas le $D_2O$, sa nature est précisée dans le texte.

Exemple 1

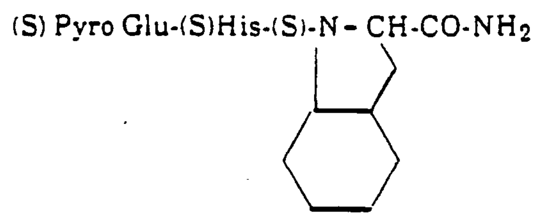

Par commodité, le groupement (2S,3aS,7aS) carbonyl-2 perhydroindole sera dénommé (S)PHI.

Stade A: tBocPHI-OH ou acide (t.butoxycarbonyl-1) (2S,3aS,7aS) perhydroindolyl-2 carboxylique

Dans un mélange de 60 ml de dioxanne et 30 ml d'eau, dissoudre 0,03 mole de (S) PHI-OH, obtenu selon Tetrahedron Letters *23* (16) 1677—1680 (1982), refroidir à 0°C, ajouter 70 ml de soude N, puis goutte à goutte une solution de 0,03 mole de carbonate de ditertiobutyle dans 150 ml de dioxanne.

Agiter pendant 30 mn à une température comprise entre 0°C et 5°C, puis 2 heures à température ambiante. Evaporer les solvants sous pression réduite. Reprendre le résidu par de l'eau acidifée par de l'acide citrique jusqu'à pH = 4 et extraire la phase aqueuse par de l'acétate d'éthyle. Laver la phase organique par une solution aqueuse de chlorure de sodium à 10%, la sécher sur sulfate de calcium anhydre, la filtrer et la concentre sous pression réduite. Cristalliser le résidu dans le n.pentane, essorer, sécher.

On obtient le tBoc(S)PHI-OH F = 14°
Rendement = 82,5%

Caractéristiques spectrales en I.R.:
ν OH: 2400—3200 $cm^{-1}$
ν CO(acide): 1750 $cm^{-1}$
ν CO(—N—CO—): 1630 $cm^{-1}$

Stade B: tBoc(S)PHI-$NH_2$

Dans une solution de 5,4 g (0,02 mole) de tBoc(S)PHI-OH dans 25 ml de tétrahydrofuranne refroidie dans un bain de glace salé (température = −10°C), on ajoute 2,10 ml (0,022 mole) de chloroformiate d'éthyle distillé fraîchement. Il se forme un précipité, on laisse agiter encore un quart d'heure à −10°C, puis on ajoute 3,84 ml (0,057 mole) d'ammoniaque concentré à la même température, on laisse encore agiter 30 mn, puis revenir à température ambiante. Evaporer les solvants sous pression réduite. Reprendre le résidu par une solution aqueuse d'acide citrique de pH = 4 et extraire la phase aqueuse acide par de l'acétate d'éthyle. Laver la phase organique par une solution aqueuse de bicarbonate de sodium, puis à l'eau, la sécher sur sulfate de calcium anhydre, filtrer et la concentrer sous pression réduite. Cristalliser le résidu dans le n.pentane, essorer, sécher.

On obtient le tBoc(S)PHI-$NH_2$
F = 163°C Rendement = 75,4%

Analyse élémentaire:

| | C% | H% | N% |
|---|---|---|---|
| Calculé | 62,66 | 9,01 | 10,43 |
| Trouvé | 62,87 | 9,02 | 10,53 |

Caractéristiques spectrales en I.R.:
ν$NH_2$ amide: 3300 à 3520 $cm^{-1}$
νCO carbonate et amide I: 1680 $cm^{-1}$
νCO amide II: 1570 $cm^{-1}$.

Stade C (S)PHI-$NH_2$

Saturer de chlorhydrique une solution de 3 g (0,0111 mole) de tBoc(S)PHI-$NH_2$ dans 150 ml d'acétate d'éthyle anhydre, abandonner 12 heures à température ambiante essorer, laver à l'acétate d'éthyle, sécher.

On obtient le (S)PHI-$NH_2$, HCl avec un rendement de 98%.

Analyse élémentaire:

| | C% | H% | N% | Cl% |
|---|---|---|---|---|
| Calculé | 52,80 | 8,37 | 13,68 | 17,31 |
| Trouvé | 53,12 | 8,50 | 13,89 | 17,20 |

Caractéristiques spectrales en I.R.:
ν$HN_2$+ (amide): 2200 à 3300 $cm^{-1}$
νCO amide (I): 1685 $cm^{-1}$.
νCo amide (II): 1580—1630 $cm^{-1}$

Stade D: tBoc(S)(2,4 DNP)His-(S)PHI-$NH_2$

En utilisant la méthode de couplage peptidique en phase liquide (DCC/HOBT) de W. KONIG et R. GEIGER (Ber. *103*, 788 (1970)) et le diméthylformamide comme solvant, on prépare à partir de 0,0128 mole de (S)PHI-$NH_2$ obtenu au stade précédent et de 0,0128 mole de tBoc (S) (dinitro-2,4 phényl) Histidine ou tBoc(S)(2,4 DNP)His, le tBoc(S)(2,4 DNP)His-(S)PHI-$NH_2$ avec un rendement de 85%.

Le produit est purifié par chromatographie sur colonne de silice en utilisant éluant le chlorure de méthylène méthanol (95/5). On obtient 2 g d'un produit dont les analyses sont conformes.

Caractéristiques spectrales en I.R.:
νNH, $NH_2$: plusieurs bandes 3100 à 3320 $cm^{-1}$
νCO amide I, amide II, C = N, plusieurs bandes 1540 à 1710 $cm^{-1}$

Stade E: (S)(2,4 DNP)His-(S)PHI-$NH_2$,HCl

Le produit obtenu au stade précédent est déprotégé par la méthode à l'acétate d'éthyle chlorhydrique décrite au stade C. En partant de 3,5 g (0,00647 mole) de tBoc(S)(2,4 DNP)His-(S)PHI-$NH_2$, on obtient 3,4 g de chlorhydrate de (S)(2,4 DNP)His-(S)PHI-$NH_2$.

Caractéristiques spectrales en I.R.:
νNH, $NH2^+$: 3580—2200 $cm^{-1}$
νCO amide (I): 1690 $cm^{-1}$
νCO amide (II): 1540 $cm^{-1}$
νCO amide tertiaire: 1640 $cm^{-1}$.

Stade F: (S)PyroGlu-(S)(2,4 DNP)His-(S)PHI-$NH_2$

En utilisant la méthode de couplage peptidique en phase liquide (DCC/HOBT), décrite au stade D, à partir de 0,0055 mole d'acide (S) pyroglutamique ou (S)PyroGlu-OH et de 0,0055 mole de (S)(2,4 DNP)His-(S)PHI-$NH_2$ obtenu au stade précédent, on obtient, après traitement usuel, un produit brut qui est chromatographié sur silice en utilisant comme éluant le mélange de chlorure de méthylène/méthanol (85/15), et récupère 1 g de produit attendu avec un rendement de 31,2%.

Caractéristiques spectrales en R.M.N. ($CDCl_3$):
0,8 à 2,5 ppm: 15H (CH et $CH_2$)
2,5 à 5 ppm: 6H ($CH_\alpha$ de CO et CN, $CH_2\alpha$ d'imidazole)
6,7 et 7,5 à 8,5 ppm: 4H échangeables (NH et $NH_2$)
7,3—8,1 ppm: 2H (CH imidazole)
7,9—8,7 et 8,9 ppm: 3H aromatiques.

Stade G: (S)PyroGlu-(S)His-(S)PHI-$NH_2$

En appliquant la méthode déprotection d'écrite par R. F. NUTT, F. W. HOLLY et al. J. Med. Chem. *24*, 692—698 (1981) à 1 g (0,0017 mole) du tripeptide obtenu au stade précédent, en solution dans 10 ml de

diméthyl formamide anhydre et agités sous atmosphère d'azote avec 1 ml (0,014 mole) de mercapto-2 éthanol pendant 14 heures, on obtient après évaporation des solvants sous pression réduite, un résidu jaune qui est chromatographié sur colonne de silice en utilisant comme éluant le mélange acétone/eau (90/10).

Le produit pur obenu est repris par de l'eau, filtré sur filtre micronique et lyophilisé.
On obtient 325 mg de (S)pyroGlu-(S)His-(S)PHI-$NH_2$ (rendement 46,5%).

Analyse élementaire: corrigée d'eau;

| | C% | H% | N% |
|---|---|---|---|
| Calculé | 57,67 | 6,77 | 20,17 |
| Trouvé | 57,46 | 6,46 | 20,02 |

La chromatographie en couche mince montre un produit monotache dans le système acétone/eau (80/20), révélable par le réactif de PAULY.

Exemple 2: (S)Blc-(S)His-(S)PHI-$NH_2$

En remplaçant dans l'exemple 1, stade F, le (S)PyroGlu-OH par l'acide (S) γ Butyrolactone carboxylique (ou acide oxo-2 tétrahydrofuranne carboxylique-5) ou (S)Blc-OH (préparé selon O. LERVINKA et L. HUB Coll. Czech. Chem. Comm., *33* 2927 (1968)), on obtient de la même façon successivement:

Stade F: (S)Blc-(S)(2,4 DNP)His-(S)PHI-$NH_2$ (rendement: 65%)
Stade G: (S)Blc-(S)His-(S)PHI-$NH_2$ (rendement: 29%) qui est lyophilisé.

Caractéristiques spectrales en I.R.:
νNH,$NH_2$, OH($H_2O$): 2500 à 3600 $cm^{-1}$
νCO lactone: 1780 $cm^{-1}$
νCO amide (I) et (II): 1630—1680 et 1530 $cm^{-1}$.

Analyse élémentaire corrigée d'eau ($H_2O$% = 4,20)

| | C% | H% | N% |
|---|---|---|---|
| Calculé | 57,54 | 6,51 | 16,77 |
| Trouvé | 57,63 | 6,27 | 16,79 |

Exemple 3: (S)Homo pyro glu-(S)His-(S)PHI-$NH_2$

En remplaçant dans l'exemple 1, stade F, le (S)PyroGlu-OH par l'acide homo pyrogluatamique (acide oxo-2 pipéridine carboxylique-6) ou (S) homo pyroglu-OH (préparé selon J. P. GREENSTEIN, S. M. BIRNBAUM J. A. C. S. *75*, 1994 (1953)), on obtient de la même façon et successivement:

Stade F: (S)Homo pyro glu-(S)(2,4 DNP)His-(S)PHI-$NH_2$ (rendement quantitatif)
Stade G: (S)Homo pyro glu-(S)His-(S)PHI-$NH_2$ (rendement 14,4%) qui est lyophilisé.

Caractéristiques spectrales en I.R.:
νNH,$NH_2$,OH($H_2O$): 3000 à 3600 $cm^{-1}$
νCO (lactame, amide primaire, amide (I)): bande large 1620—1680 $cm^{-1}$
νCO (amide (II)): 1550 $cm^{-1}$

en R.M.N.:
1 à 2,6 ppm: 17H (massif) ($CH_2$ et CH)
3 ppm: 2H ($CH_2$ en a de l'imidazole)
3,9 à 5 ppm: 4H (CHα de CO et C—N)
7 à 7,7 ppm: 2H (imidazole).

Exemple 4: Orotyl-(S)His-(S)PHI-$NH_2$

En remplaçant dans l'exemple 1, stade F, le (S)PyroGlu-OH par l'acide orotique (produit commercial), on obtient de la même façon et successivement:

Stade F: Orotyl-(S)(2,4 DNP)His-(S)PHI-$NH_2$ (rendement = 96,2%)
Stade G: Orotyl-(S)His-(S)PHI-$NH_2$ (rendement = 13%) qui est lyophilisé.

Caractéristiques spectrales en I.R.:
ν$NH_2$,NH: 2400 à 3600 $cm^{-1}$
νCO amide (I), amide (II) uracile: 1550 à 1730 $cm^{-1}$

en R.M.N.:
1 à 2,3 ppm: 11H ($CH_2$ et CH)

3,1 ppm: 2H ($CH_2$ α de imidazole)
4 à 5 ppm: 3H (CHα de CO et C—N)
6,1 ppm: 1H (CH de orotyl)
7,2 à 8,2 ppm: 2H (imidazole).

Exemple 5

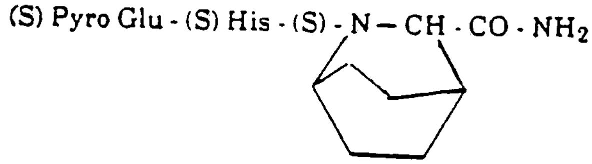

Le radical (S)carbonyl-3 aza-2 bicyclo[2,2,2]octane sera ultérieurement dénommé(S) ABO.

En remplaçant dans l'exemple 1, stade D, le (S)PHI-$NH_2$ par le (S)ABO-$NH_2$ (prépare comme aux stades A, B, C à partir de (S)ABO-OH décrit dans le brevet européen No 51020), on obtient de la même façon les composés suivants:

Stade D tBoc(2,4 DNP)His-(S)ABO-$NH_2$ (rendement = 84%)
Stade E (S)(2,4 DNP)His-(S)ABO-$NH_2$HCl (rendement = 80%)
Stade F (S)PyroGlu-(S)(2,4 DNP)His-(S)ABO-$NH_2$ (rendement = 69%)
Stade G (S)PyroGlu-(S)His-(S)ABO-$NH_2$ (rendement: 24%).
qui est transformée en son chlorhydrate par lyophilisation en présence de la quantité stoechiométrique d'acide chlorhydrique N/10.

Analyse élémentaire (corrigée de 2,9% d'$H_2O$).

| | C% | H% | N% | Cl% |
|---|---|---|---|---|
| Calculé | 51,99 | 6,20 | 19,14 | 8,07 |
| Trouvé | 51.91 | 6,50 | 18,60 | 8,05 |

Caractéristiques spectrales en I.R.:
νNH,$NH_2$,$NH^+$,OH($H_2O$): 2300 à 3600 $cm^{-1}$
νCO amide (I) et lactame: 1530 et 1570 $cm^{-1}$
νCO amide (II): 1530 $cm^{-1}$

en R.M.N.:
1,5 à 2,6 ppm: 13H ($CH_2$,CH)
3,2 ppm: 2H ($CH_2$ α d'imidazole)
4 à 5,5 ppm: 4H (CH α de CO et C—N)
7,4 et 8,7 ppm: 2H (imidazole).

Exemple 6: Orotyl-(S)His-(S)ABO-$NH_2$

En remplaçant dans l'exemple 5, stade F, l'acide (S) pyroglutamique par l'acide orotique, on obtient de la même façon et successivement:

Stade F: Orotyl (S)(2,4 DNP)His-(S)ABO-$NH_2$ (rendement: 43%)
Stade G: Orotyl (S)His-(S)ABO-$NH_2$ (rendement: 57%)
qui est transformé en son chlorhydrate par lyophilisation avec la quantité stoechiométrique d'HCl N/10.

Caractéristiques spectrales en RMN:
1,4 à 2,4 ppm: 9H (CH et $CH_2$)
3,3 ppm: 2H ($CH_2$ α d'imidazole)
3,8 à 5,5 ppm: 4H (CH en α de CO et C—N)
6,2 ppm: 1H (orotyl)
7,4 à 8,7 ppm: 2H (imidazole).

La chromatograpie couche mince montre un produit monotache acétone/eau (90/10) et révélable au réactif de PAULY.

Exemple 7: (S)Homo pyro glu-(S)His-(S)ABO-$NH_2$

En remplaçant dans l'exemple 5, stade F, l'acide (S) pyroglutamique par l'acide (S)Homo pyro glutamique, on obtient de la même façon et successivement.

Stade F: (S)HomoPyroGlu-(S)(2,4 DNP)His,-(S)ABO-$NH_2$ (rendement; 15%).
Stade G: (S)HomoPyroGlu-(S)His-(S)ABO-$NH_2$ (rendement: 53%), le produit est lyophilisé.

Analyse élémentaire (corrigée de 4,9% d'eau):

| | C% | H% | N% |
|---|---|---|---|
| Calcule: | 57,68 | 6,77 | 20,17 |
| Trouvé | 57,74 | 6,52 | 19,65 |

Caractéristiques spectrales en I.R.:
vNH,OH($H_2O$): 2600 à 3700 $cm^{-1}$
vCO (lactame-amide I, II, amide primaire, tertiaire): 1600 à 1700 $cm^{-1}$

en R.M.N:
1,3 à 2,5 ppm: 15H ($CH_2$, CH)
3 ppm: 2H ($CH_2$ α d'imidazole)
3,8 à 5,3: 4H (α de CO et C—N)
7,1—7,7 ppm: 2 (imidazole).

Exemple 8: (S)PyroGlu(S)NVa-(S)PHI-$NH_2$

En remplaçant dans l'exemple 1, stade D, tBoc-(S)(2,4 DNP)His par tBoc-(S) norvaline ou tBoc(S)NVa, on obtient de la même façon et successivement:

Stade D: tBoc(S)NVa-(S)PHI-$NH_2$ (rendement 89,7%)
Stade E: (S)NVa-(S)PHI-$NH_2$ (rendement quantitatif)
Stade F: (S)(Z)PyroGlu-(S)NVa-(S)(PHI-$NH_2$ (rendement: 54%)
Stade G: (S)PyroGlu-(S)Nva-(S)PHI—$NH_2$.

Le groupement (S) pyro glu est déprotégé par une hydrogénation catalytique dans l'éthanol en présence de charbon palladié. Après filtration du catalyseur, évaporation du solvant sous pression réduite, reprise de la résine incolore obtenue par de l'eau distillée, filtration et lyophilisation, on obtient le produit attendu avec un rendement quantitatif.

Analyse élémentaire: (corrigée de 4,6% d'eau)

| | C% | H% | N% |
|---|---|---|---|
| Calculé | 60,29 | 7,98 | 14,80 |
| Trouvé | 60,01 | 7,67 | 14,76 |

Caractéristiques spectrales en I.R.:
vNH,$H_2O$: 3700 à 3100 $cm^{-1}$
vCO amide (I): 1680—1630 $cm^{-1}$
vamide II: 1550 $cm^{-1}$
vCO amide (II): 1550 $cm^{-1}$

en R.M.N.: ($CDCl_3$)
0,9 ppm: 3H ($CH_3$)
1—2,5 ppm: 16H ($CH_2$ CH) 2H (NCO—$CH_2$)
4,1 ppm: 1H (CH α de CO—N)
4,5 ppm: 3H ($CH_\alpha$ de CO, CN)
5,4 ppm: 1H échangeables
7 ppm: 3H échangeables
$[\alpha]_D^{21,5}$ = −73,2° (valeur corrigée de 2,7% d'eau, titrée simultanément).

Exemple 9: (S)PyroGlu-(S)(méthyl-1 His)-(S)PHI-$NH_2$

En remplaçant dans l'exemple I, stade D, la tBoc(2,4 DNP)His par la tBoc(méthyl-1 His), on obtient de la même façon et successivement:

Stade D: le (S)tBoc(méthyl-1 His)-(S)PHI-$NH_2$(rendement: 83%)
Stade E: le (S)(méthyl-1 His)(S)PHI-$NH_2$ (rendement quantitatif)
Stade F: le (S)PyroGlu-(S)(méthyl-1 His)-(S)PHI-$NH_2$ (rendement: 30%)

Caractéristiques spectrales en I.R.:
vNH: 3600 à 3100 $cm^{-1}$
vCO (lactame): 1685 $cm^{-1}$
vamide I: 1630 $cm^{-1}$
vamide II: 1545 $cm^{-1}$

Exemple 10: (S)PyroGlu-(S)(méthyl-3 His)-(S)PHI-$NH_2$

En remplaçant dans l'exemple précédent la tBoc(méthyl-1 His) par la tBoc(méthyl-3 His) on obtient de la même façon:

Stade D: le (S)tBoc(méthyl-3 His)-(S)PHI-$NH_2$ (Rendement: 80%)
Stade E: le (S)(méthyl-3 His)(S)PHI-$NH_2$, (Rendement quantitatif)
Stade F: le (S)PyroGlu(S)-(méthyl-3 His)(S)-PHI-NH2 (Rendement: 30%)

Analyse élémentaire (corrigée de 3,2% d'eau)

| | C% | H% | N% |
|---|---|---|---|
| Calculé | 58.58 | 7.08 | 19.52 |
| Trouvé | 58.82 | 6.93 | 18.49 |

Caractéristiques spectrales en I.R.:
vNH,OH($H_2O$): 3700 à 3000 $cm^{-1}$
vCO (lactame): 1690 $cm^{-1}$
vamide I: 1640 $cm^{-1}$
vamide II: 1550 $cm^{-1}$.

Exemple 11: (S)pyroGlu(S)-His(S)PHI-MéPip

En remplaçant dans l'exemple I, stade B, l'ammoniaque par une quantité stoechiométrique de méthyl-4-pipérazine, ou MéPip-H, on obtient successivement et de la même façon:

Stade B: tBoc-(S)PHI-MéPip (Rendement: 48%)
Stade C: (S)PHI-MéPip (Rendement: 90%)
Stade D: tBoc-(S)(2,4 DNP)His-(S(PHI-MéPip (Rendement: 84%)
Stade E: (S)(2,4 DNP)His-(S)PHI-MéPip (Rendement quantitatif)
Stade F: (S)PyroGlu-(S)(2,4 DNP)His-(S)PHI-MéPip (Rendement: 32%)
Stage G: (S)PyroGlu-(S)His-(S)PHI-MéPip (Rendement: 47%)

Analyse élémentaire (corrigée de 6,3% d'eau)

| | C% | H% | N% |
|---|---|---|---|
| Calculé | 60.10 | 7.46 | 19.62 |
| Trouvé | 59.23 | 6.96 | 19.24 |

Caractéristiques spectrales en I.R.:
vOH($H_2O$): 3700 $cm^{-1}$
vNH: 3500 à 2400 $cm^{-1}$
vCO (lactame): 1700 $cm^{-1}$
vCO (amide): 1680 à 1600 $cm^{-1}$

en R.M.N.($CDCl_3$)
1 à 5 ppm: 32H (CH, $CH_2$ et $CH_3$) massifs
6,85 ppm: 1H (imidazole en α de la chaine) singulet
7,52 ppm: 1H (imidazole en α des 2 azotes) singulet
7,5 à 8,2 ppm: 3H (NH) échangeables par $D_2O$.

Exemple 12: (S)PyroGlu-(S)Leu(S)-PHI-Mor

En remplaçant dans l'exemple I, au stade B, l'ammoniaque par une quantité calculée de morpholine (Mor-H) et au stade D, la tBoc(S)(2,4 DNP)HIS par la tBoc(S) leucine ou tBoc(S)Leu, on obtient successivement:

Stade B: tBoc(S)PHI-Mor (Rendement: 81%)
Stade C: (S)PHI-Mor (Rendement quantitatif)
Stade D: tBoc(S)-Leu-(S)PHI-Mor (Rendement: 80%)
Stade E: (S)-Leu-(S)-PHI-Mor (Rendement quantitatif)
Stade F: (S)PyroGlu-(S)Leu-(S)PHI-Mor (Rendement: 35%)

Analyse élémentaire (corrigée de 3,5% d'eau)

| | C% | H% | N% |
|---|---|---|---|
| Calculé | 62.31 | 8.28 | 12.11 |
| Trouvé | 61.95 | 7.80 | 11.95 |

Caractéristiques spectrales en I.R.:
vCO (lactame): 1700 $cm^{-1}$
vCO (amide): 1650 à 1620 $cm^{-1}$

en. R.M.N. ($CDCl_3$):
0,9 ppm: 6H (2 $CH_3$), doublet

1 à 3 ppm: 18H (CH, $CH_2$), massif
3 à 4,2 ppm: 9H (CH, $CH_2$: morpholine; 1H PHI α de N), massif
4,2 à 5,5 ppm: 3H (CH α de CO), massif
7,4 à 8,0 ppm: 2H (NH, échangeables par $D_2O$), massif.

Exemple 13: (S)PyroGlu-(S)-NVa(S)PHI-N($C_2H_5$)$_2$

En remplaçant dans l'exemple 1, stade B, l'ammoniaque par une quantité calculé de diéthylamine, et au stade B, la tBoc(S)(2,4 DNP)His par la tBoc(S) norvaline ou tBoc(S)NVa, on obtient successivement, et de la même façon

Stade B: tBoc(S)PHI-N($C_2H_5$)$_2$ (Rendement: 96%)
Stade C: (S)PHI-N($C_2H_5$)$_2$ (Rendement quantitif)
Stade D: tBoc(S)-NVa-(S)PHI-N($C_2H_5$)$_2$ (Rendement: 62%)
Stade E: (S)NVa-(S)PHI-N($C_2H_5$)$_2$ (Rendement quantitatif)
Stade F: (S)PyroGlu-(S)NVa-(S)PHI-N($C_2H_5$)$_2$ (Rendement: 42%)

Analyse élémentaire (corrigée 1,8% d'eau)

| | C% | H% | N% |
|---|---|---|---|
| Calculé | 63.56 | 8.81 | 12.89 |
| Trouvé | 63.75 | 8.63 | 12.78 |

Caractéristiques spectrales en I.R.:
νNH et OH($H_2O$): 3700 à 2300 $cm^{-1}$
νCO (lactame et amide): 1700 à 1600 $cm^{-1}$
νCO (amide II): 1540 $cm^{-1}$.

en R.M.N. ($CDCl_3$):
0,9 ppm: 3H ($CH_3$; NVal)
1,2 ppm: 6H (2 $CH_3$; 2 $CH_2$ *$CH_3$*)
1,6 ppm: 16H ($CH_2$)
2,3 ppm: 3H (CH2; α de CO; pyrrolidone —CH β de N; PHI)
3,4 ppm: 4H (2 $CH_2$; 2 *$CH_2$*$CH_3$)
4,1 ppm: 1H (CH; α de N; PHI)
4,7 ppm: 3H (CH; α de CO)
7,4 ppm: 2H (NH échangeables par $D_2O$)

Exemple 14: (S)pyroGlu(S)-Leu-(S)PHIQ-$NH_2$

En remplaçant dans l'exemple I, stade A, le (S)PHI-OH par l'acide (S) perhydroisoquinoléïne carboxylique-3 ou (S)PHIQ-OH et au stade D la tBoc(2,4 DNP)His par la tBoc Leu, on obtient successivement:

Stade A: tBoc-(S)PHIQ-OH (Rendement: 81%)
Stade B: tBoc-(S)PHIQ-$NH_2$ (Rendement: 73%)
Stade C: (S) PHIQ-$NH_2$ (Rendement quantitatif)
Stade D: tBoc(S)Leu-(S)PHIQ-$NH_2$ (Rendement: 82%)
Stade E: (S)Leu-(S)PHIQ-$NH_2$ (Rendement quantitatif)
Stade F: (S)PyroGlu-(S)-Leu-(S)PHIQ-$NH_2$ (Rendement: 44%)

Caractéristiques spectrales en I.R.:
νNH: 3600 à 3100 $cm^{-1}$
νCO (lactame): 1700 $cm^{-1}$
νCO (amide I): 1650 à 1620 $cm^{-1}$
νCO (amide II): 1550 $cm^{-1}$

Exemple 15: (S)Blc-(S)His-(S)ABO-$NH_2$

En remplaçant dans le stade F de l'exemple 5, l'acide pyroglutamique par l'acide (S) γ butyrolactone carboxylique, on obtient de la même façon et successivement:

Stade F: (S)Blc-(S)(2,4 DNP)His-(S)ABO-$NH_2$ (Rendement 70%)
Stade G: (S)Blc-(S)His-(S)ABO-$NH_2$ (Rendement 41%)

Caractéristiques spectrales en R.M.N. ($D_2O$):
1,4 à 2,8 ppm: 13H (CH et CH2), massif
3,0 ppm: 2H (CH2; α imidazole), doublet
3,7 à 5,3 ppm: 4H (CH; α de CO et CN), massif
7 à 7,8 ppm: 2H (imidazole)

Etude Pharmacologique

Exemple 16: Stimulation de la synthèse d'AMPc cérébrale

Les composés à tester sont administrés à la dose de 10 mg/kg par voie intrapéritonéale chez la souris de souche $OF_1$/IFFA-Credo.

Cinq minutes après l'injection, les animaux sont sacrifiés par congélation, l'AMPc présent dans les structures cérébrales est dosé par radio immunologie selon la méthode de Amersham (protéine spécifique liante).

Alors que la TRH prise comme référence, augmente le contenu intracérébral d'AMPc de 46%, les composés de l'invention induisent une augmentation double (par exemple respectivement +95% et + 102% pour les composés des exemples 1 et 2).

Exemple 17: Augmentation de la latence d'apparition du sommeil induit par le Pentobarbital

Le pentobarbital, injecté chez le rat à la dose de 40 mg/kg par voie I.P., induit l'apparition du sommeil. Les composés de l'invention, administrés à la dose de 10 mg/kg par voie intrapéritonéale avant l'injection du pentobarbital, retardent l'apparition de ce sommeil (+ 110% avec le composé de l'exemple 2) prouvant ainsi une inhibition du recaptage d'acétyl choline. Dans les mêmes conditions, la TRH est inactive.

Exemple 18: Potentialisation de la toxicité de la Yohimbine

La Yohimbine, injectée chez la souris à la dose de 35 mg/kg par voie intrapéritonéale provoque la mortalité de 10 à 30% des animaux traités. Les composés de l'invention, administrés à la dose de 10 mg/kg par voie intrapéritonéale 15 minutes avant l'injection de Yohimbine augmentent le taux de mortalité (+ 80% avec les composés des exemples 5 et 7).

Dans les mêmes conditions, on note une activité identique avec la TRH.

Exemple 19: Augmentation de la motricité spontanée

Les composés de l'invention sont administrés à une dose de 30 mg/kg pr voie intrapéritonéale chez des souris placées par groupe de trois sur un enregistreur automatique de mouvementes après une heure d'exploration spontanée.

La motricité spontanée est comparée durant l'heure suivante avec celle de souris non traitées. Les composés de l'invention augmentent la motricité spontanée. Celui de l'exemple 5 exerce son activité dès la $10^{ème}$ minute suivant le traitement et son effet reste identique après 60 minutes.

Exemple de Formulation Galenique

Exemple 20: Comprimés dosés à 25 mg de (S)PyroGlu-(S)His-(S)PHI-$NH_2$

| | |
|---|---|
| —(S)PyroGlu-(S)His-(S)PHI-$NH_2$ | 25 mg |
| —Lactose | 50 mg |
| —Talc | 5 mg |
| —Amidon de maïs | 50 mg |
| —Polyvinyl pyrrolidone | 5 mg |

Pour un comprimé terminé à 135 mg.

**Revendications pour les Etats contractants: BE CH DE FR GB IT LI LU NL SE**

1. Composés de formule générale I

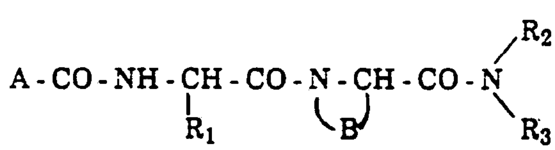

(I)

dans laquelle:

A représente un groupement oxo-2 tétrahydrofuryle-5, oxo-2 pyrrolidinyle-5 ou oxo-2 pipéridinyle-6 éventuellement substitué au niveau de l'atome d'azote par un groupement alkyle de 1 à 4 atomes de carbone, en chaîne droite ou ramifiée, ou un groupement dioxo-2,6 tétrahydro-1,2,3,6pyrimidinyle-4,

B représente, avec l'atome de carbone et l'atome d'azote auxquels il est attaché une structure polycyclique saturée choisie parmi le groupe constitué des perhydroindole, perhydroisoindole, perhydroquinoléïne, perhydroisoquinoléine, cyclopenta [b] pyrrole, et aza-2 bicyclo[2,2,2]octane,

$R_1$ représente un atome d'hydrogène, un radical alkyle renfermant de 1 à 4 atomes de carbone en chaîne droite ou ramifiée, ou un groupe (imidazolyl-4) méthyle éventuellement substitué au niveau d'un des atomes d'azote par un radical alkyle de 1 à 4 atomes de carbone en chaîne droite ou ramifiée ou par un radical benzyloxycarbonyle, dinitro-2,4 phényle, fluorénométhyloxycarbonyle, tosyle ou benzyle,

$R_2$ et $R_3$, identiques ou différents, représentent un atome d'hydrogène, un radical alkyle renfermant de 1 à 4 atomes de carbone ou forment ensemble, avec l'atome d'azote auquel ils sont attachés, un radical pyrrolidinyle-1, pipéridinyle-1, morpholinyle-4 ou alkyl (de 1 à 4 atomes de carbone)-4 pipérazinyle-1,

leurs énantiomères et diastéréoisomères,

ainsi que, lorsque $R_1$ représente un groupe(imidazolyl-4) méthyle éventuellement substitué, au niveau d'un des atomes d'azote par un radical alkyle de 1 à 4 atomes de carbone en chaîne droite ou ramifiée ou lorsque $R_2$ et $R_3$ représentent un radical N-alkyl pipérazinyle-1, leurs sels d'addition à un acide pharmaceutiquement acceptable.

2. Composés de formule générale I dans laquelle B avec l'atome d'azote et de carbone auxquels il est attaché représente une structure perhydroindole, perhydroisoquinoléïne, ou aza-2[bicyclo[2,2,2]octane, leurs énantiomères et diastéréoisomères, ainsi que, lorsque $R_1$ représente un groupe (imidazolyl-4) méthyle éventuellement substitué, ou lorsque $R_2$ et $R_3$ représentent un radical N-alkyl pipérazinyle-1, leurs sels d'addition à un acide pharmaceutiquement acceptable.

3. Composés de formule générale I dans laquelle B avec l'atome d'azote et de carbone auxquels il est attaché représente une structure perhydroindole, leurs énantiomères et diastéréoisomères ainsi que lorsque $R_1$ représente un groupe (immidoazolyl-4) méthyle éventuellement substitué, ou lorsque $R_2$ et $R_3$ représentent un radical N-alkyl pipérazinyle-1, leurs sels d'addition à un acide pharmaceutiquement acceptable.

4. Composés de formule générale I dans laquelle B avec l'atome d'azote et de carbone auxquels il est attaché représente une structure aza-2 bicyclo[2,2,2]octane, leurs énantiomères et diastéréoisomères, ainsi que lorsque $R_1$ représente un groupe (imidazolyl-4) méthyle éventuellement substitué, ou lorsque $R_2$ et $R_3$ représentent un radical N-alkyl pipérazinyle-1, leurs sels d'addition à un acide pharmaceutiquement acceptable.

5. Le Pyro Glu-His-PHI-$NH_2$, ses énantiomères ou diastéréoisomères, ainsi que ses sels d'addition à un acide pharmaceutiquement acceptable, PHI représentant le radical carbonyl-2 perhydroindole.

6. Le Blc-His-PHI-$NH_2$, ses énantiomères ou diastéréoisomères, ainsi que ses sels d'addition à un acide pharmaceutiquement acceptable, PHI représentant le radical carbonyl-2 perhydroindole.

7. Le Pyro Glu-Nva-PHI-$NH_2$, ses énantiomères ou diastéréoisomères, PHI représentant le radical carbonyl-2perhydroindole.

8. Le Pyro Glu-His-ABO-$NH_2$, ses énantiomères ou diastéréoisomères, ainsi que ses sels d'addition à un acide pharmaceutiquement acceptable, ABO représentant un radical carbonyl-3 aza-2 bicyclo[2,2,2]octane.

9. L'Homo-Pyro Glu-His-ABO-$NH_2$, ses énantiomères ou diastéréoisomères, ainsi que ses sels d'addition à un acide pharmaceutiquement acceptable, ABO représentant un radical carbonyl-3 aza-2 bicyclo[2,2,2]octane.

10. Le Blc-His-ABO-$NH_2$, ses énantiomères ou diastéréoisomères, ainsi que ses sels d'addition à un acide pharmaceutiquement acceptable, ABO représentant un radical carbonyl-3 aza-2 bicyclo[2,2,2]octane.

11. Procédé de préparation des dérivés de formule I, caractérisé en ce que l'on protège la fonction aminée d'un amino acide de formule II

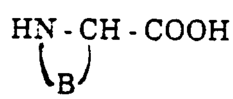

(II)

dans laquelle B avec les atomes d'azote et de carbone auxquels il est attaché a la même signification que dans la formule I, par un radical tertiobutoxy carbonyle (tBoc) sous l'action du carbonate de ditertiobutyle, en un dérivé de formule III

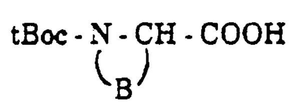

(III)

dans laquelle B a la même signification que dans la formule I,

qui est ensuite transformé, à une température comprise entre 0 et −15°C, par action successive du chloroformiate d'éthyle et d'une amine de formule IV

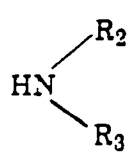

(IV)

dans laquelle $R_2$, et $R_3$ ont les mêmes significations que dans la formule I, en un amide de formule V

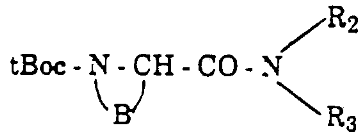

(V)

dans laquelle B, $R_2$ et $R_3$ ont les mêmes significations que dans la formule I, que l'on déprotège par action de l'acide chlorhydrique gazeux dans l'acétate d'éthyle ou de l'acide trifluoroacétique en un dérivé de formule VI

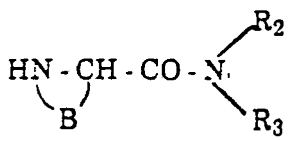

(VI)

dans laquelle B, $R_2$, $R_3$ ont les mêmes significations que dans la formule I, qui est couplé avec un amino acide protégé de formule VII,

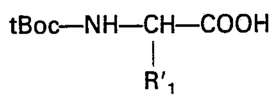

(VII)

dans laquelle $R'_1$ représente un atome d'hydrogène, un radical alkyle de 1 à 4 atomes de carbone en chaîne droite ou ramifiée, un radical (N-alkyl (de $C_1$ à $C_4$)imidazolyl)-4 méthyle ou un radical imidazolyl-4 méthyle préalablement protégé au niveau d'un des atomes d'azote par un groupement tel qu'un radical dinitro-2,4 phényle, benzyloxycarbonyle, fluorénométhyloxy carbonyle, tosyle, ou benzyle, en un dérivé de formule VIII,

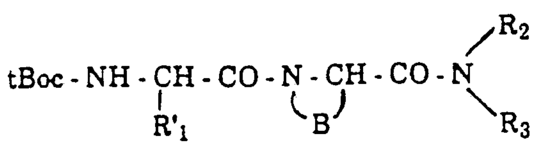

(VIII)

dans laquelle $R'_1$, $R_2$, $R_3$ et B ont les mêmes significations définies précédemment, que l'on déprotège comme précédemment en un dérivé de formule IX,

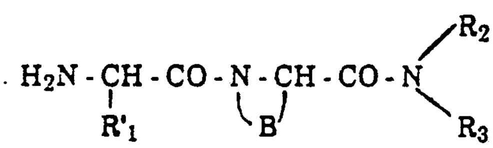

(IX)

dans laquelle $R'_1$, $R_2$, $R_3$ et B ont les mêmes significations que précédemment, qui est condensé avec un dérivé de formule X,

A—COOH (X)

dans laquelle A a les mêmes significations que dans la formule I, *soit en un dérivé de formule I',

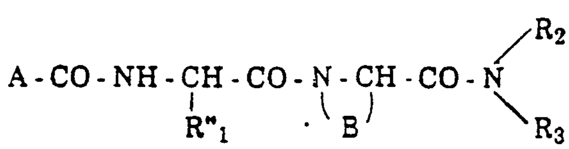

(I')

dans laquelle A, B, $R_2$, $R_3$ ont les mêmes significations que précédemment et $R''_1$ représente, un atome d'hydrogène, un radical alkyle de 1 à 4 atomes de carbone en chaîne droite ou ramifiée ou un radical (N-alkyl(de 1 à 4 atomes de carbone)imidazolyl-4)méthyle,
qui est si l'on désire transformé directement en son sel d'addition à un acide pharmaceutiquement acceptable lorsque $R''_1$ représente un radical (N-alkyl imidazolyl) méthyle ou lorsque $R_2$ et $R_3$ représentent, avec l'atome d'azote auxquels ils sont attachés, un radical pipérazinyl-1 substitué par une chaîne alkyle de 1 à 4 atomes de carbone
ou au préalable séparé en ses énantiomères ou diastéréoisomères, puis transformé si l'on désire, lorsque $R''_1$, $R_2$ et $R_3$ ont les mêmes significations que ci-dessus définies en son sel d'addition à un acide pharmaceutiquement acceptable,
*soit en un dérivé de formule I'':

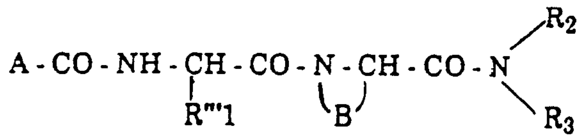

(I'')

dans laquelle A, B, $R_2$ et $R_3$ ont les mêmes significations que précédemment et $R'''_1$ représente un groupement imidazolyl-4 méthyle protégé au niveau d'un des atomes d'azote par un groupement, dinitro-2,4-phényle, benzyloxycarbonyle, fluorénométhyloxycarbonyle, tosyle, ou benzyle,
que l'on déprotège en un dérivé de formule I''',

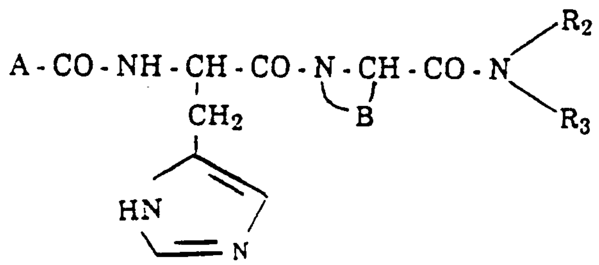

(I''')

dans lequel A, B, $R_2$ et $R_3$ gardent les significations ci-dessus définies,
qui est si l'on désire transformé en son sel d'addition à un acide pharmaceutiquement acceptable,
ou séparé en ses isomères puis si nécessaire salifié par un acide pharmaceutiquement acceptable.

12. Composition pharmaceutique contenant comme principe actif au moins un composé selon l'une quelconque des revendications 1 à 10, seul ou en combinaison avec un ou plusieurs excipients ou véhicules inertes, non toxiques, pharmaceutiquement acceptables.

13. Composition pharmaceutique selon la revendication 12 utile pour le traitement des troubles de la sénescence, liés au vieillissement normale ou pathologique et des troubles du système nerveux central.

**Revendication pour l'Etat contractant: AT**

Procédé de préparation de composés de formule générale I

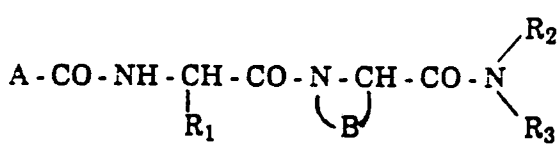

(I)

dans laquelle:

A représente un groupement oxo-2 tétrahydrofuryle-5, oxo-2 pyrrolidinyle-5 ou oxo-2 pipéridinyle-6 éventuellement substitué au niveau de l'atome d'azote par un groupement alkyle de 1 à 4 atomes de carbone, en chaîne droite ou ramifiée, ou un groupement dioxo-2,6 tétrahydro-1,2,3,6pyrimidinyle-4,

B représente, avec l'atome de carbone et l'atome d'azote auxquels il est attaché une structure polycyclique saturée choisie parmi le groupe constitué des perhydroindole, perhydroisoindole, perhydroquinoléïne, perhydroisoquinoléïne, cyclopenta [b] pyrrole, et aza-2 bicyclo[2,2,2]octane,

$R_1$ représente un atome d'hydrogène, un radical alkyle renfermant de 1 à 4 atomes de carbone en chaîne droite ou ramifiée, ou un groupe (imidazolyl-4) méthyle éventuellement substitué au niveau d'un des atomes d'azote par un radical alkyle de 1 à 4 atomes de carbone en chaîne droite ou ramifiée ou par un radical benzyloxycarbonyle, dinitro-2,4 phényle, fluorénométhyloxycarbonyle, tosyle ou benzyle,

$R_2$ et $R_3$, identiques ou différents, représentent un atome d'hydrogène, un radical alkyle renfermant de 1 à 4 atomes de carbone ou forment ensemble, avec l'atome d'azote auquel ils sont attachés, un radical pyrrolidinyle-1, pipéridinyle-1, morpholinyle-4 ou alkyl (de 1 à 4 atomes de carbone)-4 pipérazinyle-1,

leurs énantiomères et diastéréoisomères,

ainsi que, lorsque $R_1$ représente un groupe(imidazolyl-4) méthyle éventuellement substitué, au niveau d'un des atomes d'azote par un radical alkyle de 1 à 4 atomes de carbone en chaîne droite ou ramifiée ou lorsque $R_2$ et $R_3$ représentent un radical N-alkyl pipérazinyle-1, leurs sels d'addition à un acide pharmaceutiquement acceptable, caractérisé en ce que l'on protète la fonction aminée d'un amino acide de formule II

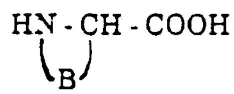

(II)

dans laquelle B avec les atomes d'azote et de carbone auxquels il est attaché a la même signification que dans la formule I, par un radical tertiobutoxy carbonyle (tBoc) sous l'action du carbonate de ditertiobutyle, en un dérivé de formule III

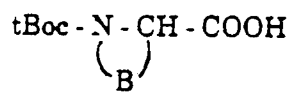

(III)

dans laquelle B a la même signification que dans la formule I,

qui est ensuite transformé, à une température comprise entre 0 et −15°C, par action successive du chloroformiate d'éthyle et d'une amine de formule IV

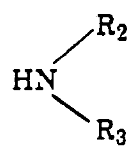

(IV)

dans laquelle $R_2$, et $R_3$ ont les mêmes significations que dans la formule I,

en un amide de formule V

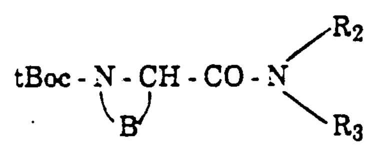

(V)

dans laquelle B, $R_2$ et $R_3$ ont les mêmes significations que dans la formule I,

que l'on déprotège par action de l'acide chlorhydrique gazeux dans l'acétate d'éthyle ou de l'acide trifluoroacétique en un dérivé de formule VI

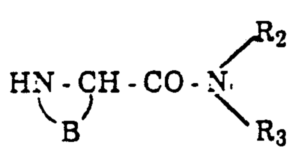

(VI)

dans laquelle B, $R_2$, $R_3$ ont les mêmes significations que dans la formule I, qui est couplé avec un amino acide protégé de formule VII,

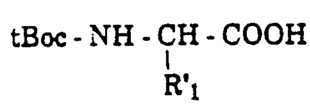

(VII)

dans laquelle $R'_1$ représente un atome d'hydrogène, un radical alkyle de 1 à 4 atomes de carbone en chaîne droite ou ramifiée, un radical(N-alkyl (de $C_1$ à $C_4$)imidazolyl)-4 méthyle, ou un radical imidazolyl-4 méthyle préalablement protégé au niveau d'un des atomes d'azote par un groupement tel qu'un radical dinitro-2,4 phényle, benzyloxycarbonyle, fluorénométhyloxy carbonyle, tosyle, ou benzyle,

en un dérivé de formule VIII,

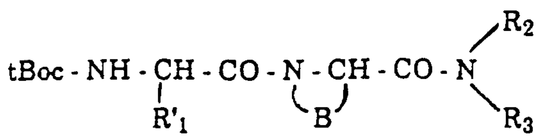

(VIII)

dans laquelle $R'_1$, $R_2$, $R_3$ et B ont les mêmes significations définies précédemment, que l'on déprotège comme précédemment en un dérivé de formule IX,

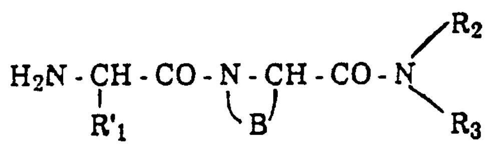

(IX)

dans laquelle $R'_1$, $R_2$, $R_3$ et B ont les mêmes significations que précédemment, qui est condensé avec un dérivé de formule X,

A—COOH (X)

dans laquelle A a les mêmes significations que dans la formule I,
*soit en un dérivé de formule I',

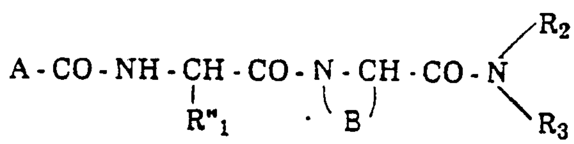

(I')

dans laquelle A, B, $R_2$, $R_3$ ont les mêmes significations que précédemment et $R''_1$ représente, un atome d'hydrogène, un radical alkyle de 1 à 4 atomes de carbone en chaîne droite ou ramifiée ou un radical (N-alkyl(de 1 à 4 atomes de carbone)imidazolyl-4)méthyle,
qui est si l'on désire transformé directement en son sel d'addition à un acide pharmaceutiquement acceptable lorsque $R''_1$ représente un radical (N-alkyl imidazolyl) méthyle ou lorsque $R_1$ et $R_2$ représentent, avec l'atome d'azote auxquels ils sont attachés un radical pipérazinyl-1 substitué par une chaîne alkyle de 1 à 4 atomes de carbone
ou au préalable séparé en ses énantiomères ou diastéréoisomères, puis transformé si l'on désire, lorsque $R''_1$, $R_2$ et $R_3$ ont les mêmes significations que ci-dessus définies en son sel d'addition à un acide pharmaceutiquement acceptable,
*soit en un dérivé de formule I'':

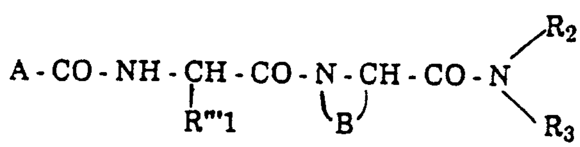

(I'')

dans laquelle A, B, $R_2$ et $R_3$ ont les mêmes significations que précédemment et $R'''_1$ représente un groupement imidazolyl-4 méthyle protégé au niveau d'un des atomes d'azote par un groupement, dinitro-2,4-phényle, benzyloxycarbonyle, fluorénométhyloxycarbonyle, tosyle, ou benzyle,
que l'on déprotège en un dérivé de formule I''',

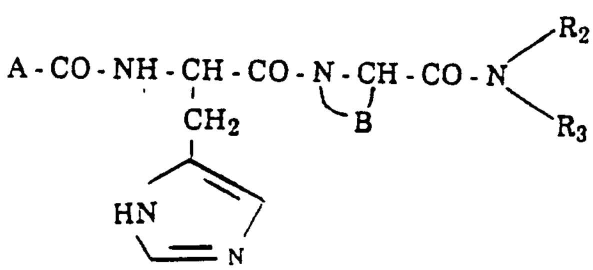

(I''')

dans lequel A, B, $R_2$ et $R_3$ gardent les significations ci-dessus définies,
qui est si l'on désire transformé en son sel d'addition à un acide pharmaceutiquement acceptable,
ou séparé en ses isomères puis si nécessaire salifié par un acide pharmaceutiquement acceptable.

**Patentansprüche für die Vertragsstaaten: BE CH DE FR GB IT LI LU NL SE**

1. Verbindungen der allgemeinen Formel (I)

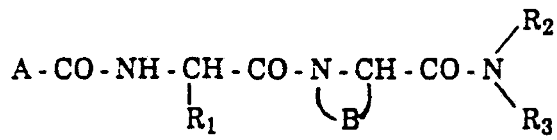

(I)

in der

A eine 2-Oxo-5-tetrahydrofuryl-, 2-Oxo-5-pyrrolidinyl- oder 2-Oxo-6-piperidinyl-Gruppe, die gegebenenfalls am Stickstoffatom durch eine geradkettig oder verzweigte Alkylgruppe mit 1 bis 4 Kohlenstoffatomen substituiert ist, oder eine 2,6-Dioxo-1,2,3,6-tetrahydro-4-pirimidinyl-Gruppe bedeutet,

B zusammen mit dem Kohlenstoffatom und dem Stickstoff, an welche er gebunden ist, einen gesättigten polycyclischen Rest darstellt, der ausgewählt ist aus der Gruppe, die Perhydroindol-, Perhydroisoindol-, Perhydrochinolin-, Perhydroisochinolin-, Cyclopenta[b]pyrrol und 2-Aza-bicyclo[2,2,2]octan-Reste umfaßt,

$R_1$ ein Wasserstoffatom, eine geradkettige oder verzweigte Alkylgruppe mit 1 bis 4 Kohlenstoffatomen oder eine (4-Imidazolyl)-methyl-Gruppe, die gegebenenfalls an einem der Stickstoffatome durch eine geradkettige oder verzweigte Alkylgruppe mit 1 bis 4 Kohlenstoffatomen oder eine Benzyloxycarbonylgruppe, eine 2,4-Dinitrophenylgruppe, eine Fluorenomethyloxycarbonylgruppe, eine Tosylgruppe oder eine Benzylgruppe substituiert ist, bedeutet,

$R_2$ und $R_3$, die gleichartig oder verschieden sein können, Wasserstoffatome, Alkylgruppen mit 1 bis 4 Kohlenstoffatomen oder gemeinsam mit dem Stickstoffatom, an das sie gebunden sind, einen 1-Pyrrolidinyl-, 1-Piperidinyl-, 4-Morpholinyl- oder 4-Alkyl-(mit 1 bis 4 Kohlenstoffatomen)-1-piperazinyl-Rest bedeuten,

deren Enantiomeren und Diastereoisomere sowie, wenn $R_1$ eine (4-Imidazolyl)-methylgruppe, die gegebenenfalls an einem der Stickstoffatome durch eine geradkettige oder verzweigte Alkylgruppe mit 1 bis 4 Kohlenstoffatomen substituiert ist, bedeutet, oder wenn $R_2$ und $R_3$ einen N-alkyl-1-piperazinyl-Rest bedeuten, deren Additionssalze mit einer pharmazeutisch annehmbaren Säure.

2. Verbindungen der allgemeinen Formel I, in der B zusammen mit dem Stickstoffatom und dem Kohlenstoffatom, an die er gebunden ist, einen Perhydroindol-, Perhydroisochinolin- oder 2-Aza-bicyclo[2,2,2]octan-Rest bedeutet, deren Enantiomere und Diastereoisomere sowie, wenn $R_1$ eine gegebenenfalls substituierte (4-Imidazolyl)-methylgruppe bedeutet oder wenn $R_2$ und $R_3$ einen N-alkyl-piperazinyl-Rest bedeuten, deren Additionssalze mit einer pharmazeutisch annehmbaren Säure.

3. Verbindungen der allgemeinen Formel I, in der B zusammen mit dem Stickstoffatom und dem Kohlenstoffatom, an den er gebunden ist, einen Perhydroindolrest bedeutet, deren Enantiomere und Diastereoisomere sowie, wenn $R_1$ eine gegebenenfalls substituierte (4-Imidazolyl)-methylgruppe bedeutet oder wenn $R_2$ und $R_3$ einen N-akyl-1-piperazinyl-Rest bedeuten, deren Additionssalze mit einer pharmazeutisch annehmbaren Säure.

4. Verbindungen der allgemeinen Formel I, in der B zusammen mit dem Stickstoffatom und dem Kohlenstoffatom, an den er gebunden ist, einen 2-Aza-bicyclo[2,2,2]octan-Rest bedeutet, deren Enantiomere und Diastereoisomere sowie, wenn $R_1$ eine gegebenenfalls substituierte (4-Imidazolyl)-methylgruppe bedeutet oder wenn $R_2$ und $R_3$ einen N-alkyl-1-piperazinyl-Rest bedeuten, deren Additionssalze mit einer pharmazeutisch annehmbaren Säure.

5. Pyro-Glu-His-PHI-$NH_2$, dessen Enantiomere oder Diastereoisomere sowie dessen Additionssalze mit einer pharmazeutisch annehmbaren Säure, worin PHI den 2-Carbonyl-perhydroindolrest bedeutet.

6. Blc-His-PHI-$NH_2$ dessen Enantiomere oder Diastereoisomere sowie dessen Additionssalze mit einer pharmazeutisch annehmbaren Säure, worin PHI den 2-Carbonyl-perhydroindolrest bedeutet.

7. Pyro-Glu-Nva-PHI-$NH_2$, dessen Enantiomere oder Diastereoisomere, worin PHI den 2-Carbonyl-perhydroindolrest bedeutet.

8. Pyro-Glu-His-ABO-$NH_2$, dessen Enantiomere oder Diastereoisomere sowie dessen Additionssalze mit einer pharmazeutisch annehmbaren Säure, worin ABO für einen 3-Carbonyl-2-aza-bicyclo[2,2,2]octan-Rest steht.

9. Homo-Pyro-Glu-His-ABO-$NH_2$, dessen Enantiomere oder Diastereoisomere sowie dessen Additionssalze mit einer pharmazeutisch annehmbaren Säure, worin ABO für einen 3-Carbonyl-2-aza-bicyclo[2,2,2]octan-Rest steht.

10. Blc-His-ABO-$NH_2$, dessen Enantiomere oder Diastereoisomere sowie dessen Additionssalze mit einer pharmazeutisch annehmbaren Säure, worin ABO für einen 3-Carbonyl-2-aza-bicyclo[2,2,2]octan-Rest steht.

11. Verfahren zur Herstellung der Verbindungen der Formel I, dadurch gekennzeichnet, daß man die Aminogruppe einer Aminosäure der Formel II

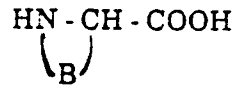

(II)

in der B zusammen mit dem Stickstoffatom und dem Kohlenstoffatom, an die er gebunden ist, die gleiche Bedeutung besitzt, wie sie für die Formel I angegeben ist, durch Umsetzten mit Di-tert.-butylcarbonat mit einem tert.-Butoxycarbonylrest (tBoc) schützt unter Bildung eines Derivats der Formel III

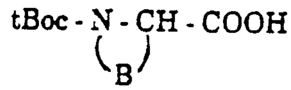

(III)

in der B die gleichen Bedeutungen besitzt wie bezüglich der Formel I angegeben, welches anschließend bei einer Temperatur zwischen 0 und −15°C durch aufeinander folgende Umsetzung mit Chlorameisensäureethylester und einem Amin der Formel IV

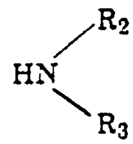

(IV)

in der $R_2$ und $R_3$ die gleichen Bedeutungen besitzen, wie sie für die Formel I angegeben sind, in ein Amid der Formel V

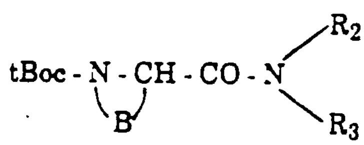

(V)

in der B, $R_2$ und $R_3$ die gleichen Bedeutungen besitzen, wie sie für die Formel I angegeben sind, umgewandelt wird,
dessen Schutzgruppen man durch Einwirkung von gasförmiger Chlorwasserstoffsäure in Ethylacetat oder durch Triofluoressigsäure abspaltet unter Bildung eines Derivats der Formel VI

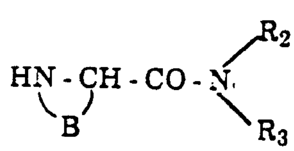

(VI)

in der B, $R_2$ und $R_3$ die gleichen Bedeutungen bestizen, wie sie für die Formel I angegeben sind, welches mit einer geschützten Aminosäure der Formel VII

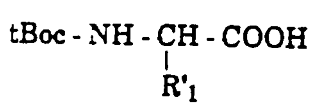

(VII)

worin $R'_1$ ein Wasserstoffatom, eine geradkettige oder verzweigte Alkylgruppe mit 1 bis 4 Kohlenstoffatomen, eine 4-(N—$C_1$—$C_4$-Alkyl-imidazolyl)-methylgruppe oder eine 4-Imidazolylmethylgruppe, die zuvor im Bereich einzelner Stickstoffatome durch eine 2,4-Dinitro-phenylgruppe, Benzyloxycarbonylgruppe, Fluorenomethyloxycarbonylgruppe, Tosylgruppe oder Benzylgruppe geschützt worden ist, zu einem Derivat der Formel VIII

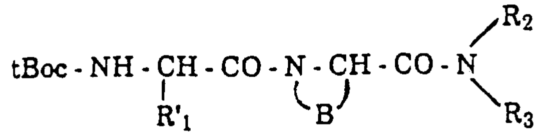

(VIII)

in der $R'_1$, $R_2$ und $R_3$ und B die oben angegebenen Bedeutungen besitzen, gekuppelt wird, dessen Schutzgruppen man wie oben angegeben abspaltet unter Bildung eines Derivats der Formel IX

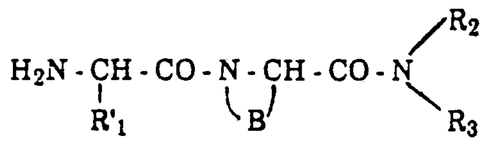

(IX)

in der $R'_1$, $R_2$, $R_3$ und B die oben angegebenen Bedeutungen besitzen,
welches man mit einem Derivat der Formel X

A—COOH (X)

in der A die gleichen Bedeutungen besitzt wie für die Formel I angegeben,
*entweder zu einem Derivat der Formel I′

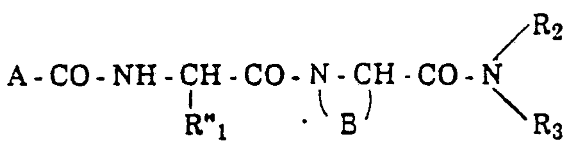

(I′)

in der A, B, $R_2$ und $R_3$ die oben angegebenen Bedeutungen besitzen und $R''_1$ ein Wasserstoffatom, eine geradkettige oder verzweigte Alkylgruppe mit 1 bis 4 Kohlenstoffatomen oder eine (N-Alkyl(mit 1 bis 4 Kohlenstoffatomen)-4-imidazolyl)-methyl-Gruppe bedeutet,
welches man, wenn $R''_1$ einen (N-Alkyl-imidazolyl)-methylrest bedeutet oder wenn $R_2$ und $R_3$ zusammen mit dem Stickstoffatom, an das sie gebunden sind, einen mit einer Alkylkette mit 1 bis 4 Kohlenstoffatomen substituierten 1-Piperazinyl-Rest bedeuten, gewünschtenfalls direkt in sein Additionssalz mit einer pharmazeutisch annehmbaren Säure umwandelt,
oder zuvor in die Enantiomeren oder Diastereoisomeren trennt und dann, wen $R''_1$, $R_2$ und $R_3$ die oben angegebenen Bedeutungen besitzen, in sein Additionssalz mit einer pharmazeutisch annehmbaren Säure umwandelt, oder
*zu einem Derivat der Formel I″

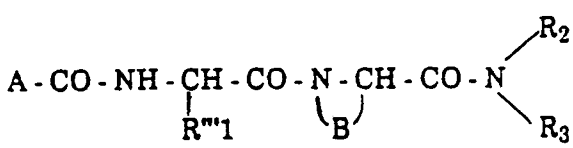

(I″)

kondensiert, in der A, B, $R_2$ und $R_3$ die oben angegebenen Bedeutungen besitzen und $R'''_1$ eine im Bereich eines der Stickstoffatome durch eine 2,4-Dinitro-phenylgruppe, Benzyloxycarbonylgruppe, Fluorenomethyloxycarbonylgruppe, Tosylgruppe oder Benzylgruppe geschützte 4-Imidazolylmethylgruppe bedeutet,
dessen Schutzgruppen man unter Bildung eines Derivats der Formel I‴

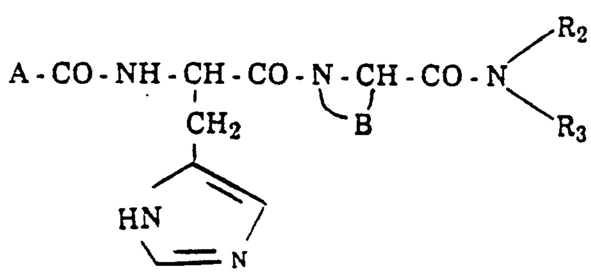

(I‴)

worin A, B, $R_2$ und $R_3$ die oben angegebenen Bedeutungen besitzen, abspaltet,
welches man gewünschtenfalls in sein Additionssalz mit eine pharmazeutisch annehmbaren Säure umwandelt oder

in seine Isomeren auftrennt und diese anschließend erforderlichenfalls mit einer pharmazeutisch annehmbaren Säure in die Salze überführt.

12. Pharmazeutische Zubereitung enthaltend als Wirkstoff mindestens eine Verbindung nach einem der Ansprüche 1 bis 10 allein oder in Kombination mit einem oder mehreren inerten, nicht-toxischen, pharmazeutisch annehmbaren Trägermaterialien oder Bindemitteln.

13. Pharmazeutische Zubereitung nach Anspruch 12 zur Behandlung von mit dem normalen oder dem pathologischen Altern auftretenden Störungen des Alterns und Störungen des zentralen Nervensystems.

**Patentanspruch für den Vertragsstaat: AT**

Verfahren zur Herstellung von Verbindungen der allgemeinen Formel I

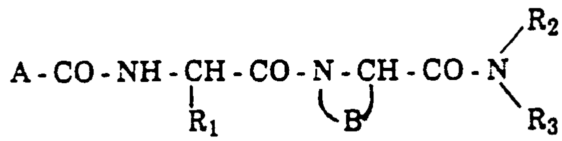

(I)

in der

A eine 2-Oxo-5-tetrahydrofuryl-, 2-Oxo-5-pyrrolidinyl- oder 2-Oxo-6-piperidinyl-Gruppe, die gegebenenfalls am Stickstoffatom durch eine geradkettig oder verzweigte Alkylgruppe mit 1 bis 4 Kohlenstoffatomen substituiert ist, oder eine 2,6-Dioxo-1,2,3,6-tetrahydro-4-pirimidinyl-Gruppe bedeutet,

B zusammen mit dem Kohlenstoffatom und dem Stickstoff, an welche er gebunden ist, einen gesättigten polycyclischen Rest darstellt, der ausgewählt ist aus der Gruppe, die Perhydroindol-, Perhydroisoindol-, Perhydrochinolin-, Perhydroisochinolin-, Cyclopenta[b]pyrrol und 2-Aza-bicyclo[2,2,2]octan-Reste umfaßt,

$R_1$ ein Wasserstoffatom, eine geradkettige oder verzweigte Alkylgruppe mit 1 bis 4 Kohlenstoffatomen oder eine (4-Imidazolyl)-methyl-Gruppe, die gegebenenfalls an einem der Stickstoffatome durch eine geradkettige oder verzweigte Alkylgruppe mit 1 bis 4 Kohlenstoffatomen oder eine Benzyloxycarbonylgruppe, eine 2,4-Dinitrophenylgruppe, eine Fluorenomethyloxycarbonylgruppe, eine Tosylgruppe oder eine Benzylgruppe substituiert ist, bedeutet,

$R_2$ und $R_3$, die gleichartig oder verschieden sein können, Wasserstoffatome, Alkylgruppen mit 1 bis 4 Kohlenstoffatomen oder gemeinsam mit dem Stickstoffatom, an das sie gebunden sind, einen 1-Pyrrolidinyl-, 1-Piperidinyl-, 4-Morpholinyl- oder 4-Alkyl-(mit 1 bis 4 Kohlenstoffatomen)-1-piperazinyl-Rest bedeuten,

von deren Enantiomeren und Diastereoisomeren sowie, wenn $R_1$ eine (4-Imidazolyl)-methylgruppe, die gegebenenfalls an einem der Stickstoffatome durch eine geradkettige oder verzweigte Alkylgruppe mit 1 bis 4 Kohlenstoffatomen substituiert ist, bedeutet, oder wenn $R_2$ und $R_3$ einen N-alkyl-1-piperazinyl-Rest bedeuten, von deren Additionssalzen mit einer pharmazeutisch annehmbaren Säure, dadurch gekennzeichnet, daß man die Aminogruppe einer Aminosäure der Formel II

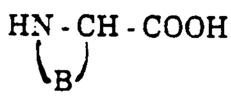

(II)

in der B zusammen mit dem Stickstoffatom und dem Kohlenstoffatom, an die er gebunden ist, die gleiche Bedeutung besitzt, wie sie für die Formel I angegeben ist, durch Umsetzten mit Di-tert.-butylcarbonat mit einem tert.-Butoxycarbonylrest (tBoc) schützt unter Bildung eines Derivats der Formel III

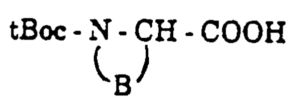

(III)

in der B die gleichen Bedeutungen besitzt wie bezüglich der Formel I angegeben, welches anschließend bei einer Temperatur zwischen 0 und −15°C durch aufeinander folgende Umsetzung mit Chlorameisensäureethylester und einem Amin der Formel IV

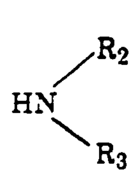

(IV)

in der $R_2$ und $R_3$ die gleichen Bedeutungen besitzen, wie sie für die Formel I angegeben sind, in ein Amid der Formel V

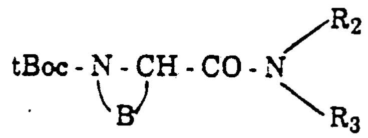

(V)

in der B, $R_2$ und $R_3$ die gleichen Bedeutungen besitzen, wie sie für die Formel I angegeben sind, umgewandelt wird,
dessen Schutzgruppen man durch Einwirkung von gasförmiger Chlorwasserstoffsäure in Ethylacetat oder durch Triofluoressigsäure abspaltet unter Bildung eines Derivats der Formel VI

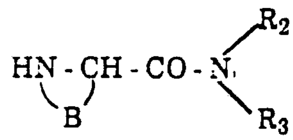

(VI)

in der B, $R_2$ und $R_3$ die gleichen Bedeutungen bestizen, wie sie für die Formel I angegeben sind, welches mit einer geschützten Aminosäure der Formel VII

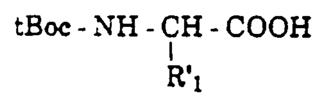

(VII)

worin $R'_1$ ein Wasserstoffatom, eine geradkettige oder verzweigte Alkylgruppe mit 1 bis 4 Kohlenstoffatomen, eine 4-(N—$C_1$—$C_4$-Alkyl-imidazolyl)-methylgruppe oder eine 4-Imidazolylmethylgruppe, die zuvor im Bereich einzelner Stickstoffatome durch eine 2,4-Dinitro-phenylgruppe, Benzyloxycarbonylgruppe, Fluorenomethyloxycarbonylgruppe, Tosylgruppe oder Benzylgruppe geschützt worden ist, zu einem Derivat der Formel VIII

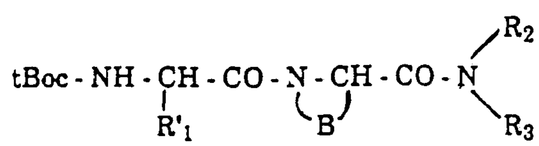

(VIII)

in der $R'_1$, $R_2$ und $R_3$ und B die oben angegebenen Bedeutungen besitzen, gekuppelt sind, dessen Schutzgruppen man wie oben angegeben abspaltet unter Bildung eines Derivats der Formel IX

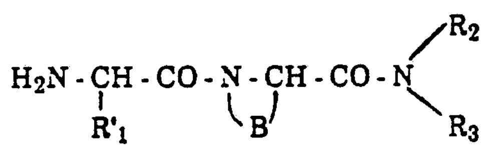

(IX)

in der $R'_1$, $R_2$, $R_3$ und B die oben angegebenen Bedeutungen besitzen,
welches man mit einem Derivat der Formel X

A—COOH (X)

in der A die gleichen Bedeutungen besitzt wie für die Formel I angegeben,
*entweder zu einem Derivat der Formel I′

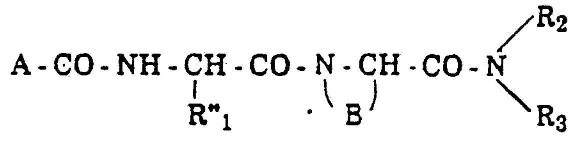

(I′)

in der A, B, $R_2$ und $R_3$ die oben angegebenen Bedeutungen besitzen und $R''_1$ ein Wasserstoffatom, eine geradkettige oder verzweigte Alkylgruppe mit 1 bis 4 Kohlenstoffatomen oder eine (N-Alkyl(mit 1 bis 4 Kohlenstoffatomen)-4-imidazolyl)-methyl-Gruppe bedeutet,
welches man, wenn $R''_1$ einen (N-Alkyl-imidazolyl)-methylrest bedeutet oder wenn $R_2$ und $R_3$ zusammen

mit dem Stickstoffatom, an das sie gebunden sind, einen mit einer Alkylkette mit 1 bis 4 Kohlenstoffatomen substituierten 1-Piperazinyl-Rest bedeuten, gewünschtenfalls direkt in sein Additionssalz mit einer pharmazeutisch annehmbaren Säure umwandelt,
oder zuvor in die Enantiomeren oder Diastereoisomeren trennt und dann, wen $R''_1$, $R_2$ und $R_3$ die oben angegebenen Bedeutungen besitzen, in sein Additionssalz mit einer pharmazeutisch annehmbaren Säure umwandelt, oder
*zu einem Derivat der Formel I''

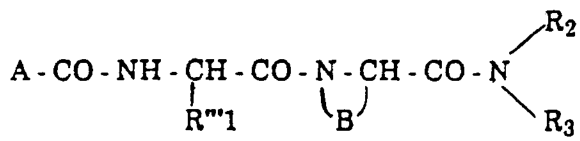
(I'')

kondensiert, in der A, B, $R_2$ und $R_3$ die oben angegebenen Bedeutungen besitzen und $R'''_1$ eine im Bereich eines der Stickstoffatome durch eine 2,4-Dinitro-phenylgruppe, Benzyloxycarbonylgruppe, Fluorenomethyloxycarbonylgruppe, Tosylgruppe oder Benzylgruppe geschützte 4-Imidazolylmethylgruppe bedeutet,
dessen Schutzgruppen man unter Bildung eines Derivats der Formel I'''

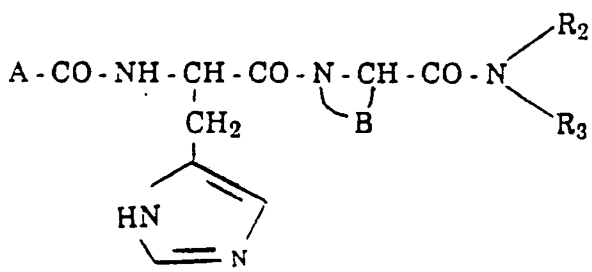
(I''')

worin A, B, $R_2$ und $R_3$ die oben angegebenen Bedeutungen besitzen, abspaltet,
welches man gewünschtenfalls in sein Additionssalz mit eine pharmazeutisch annehmbaren Säure umwandelt oder
in seine Isomeren auftrennt und diese anschließend erforderlichenfalls mit einer pharmazeutisch annehmbaren Säure in die Salze überführt.

**Claims for the Contracting States: BE CH DE FR GB IT LI LU NL SE**

1. Compounds of the general formula I

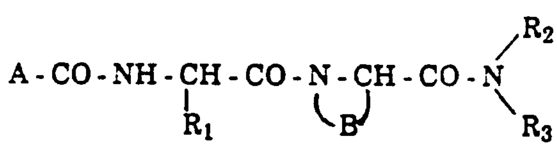
(I)

in which

A represents a 2-oxo-5-tetrahydofuryl, 2-oxo-5-pyrrolidinyl or 2-oxo-6-piperidinyl grouping optionally substituted at the nitrogen atom by a straight-chain or branched alkyl grouping having from 1 to 4 carbon atoms, or represents a 2,6-dioxo-1,2,3,6-tetrahydro-4-pyrimidinyl grouping,

B represents, together with the carbon and nitrogen atom to which it is attached, a saturated polycyclic structure selected from the group comprising perhydroindole, perhydroisoindole, perhydroquinoline, perhydroisoquinoline, cyclopenta[b]pyrrole and 2-azabicyclo[2,2,2]octane,

$R_1$ represents a hydrogen atom, a straight-chain or branched alkyl radical having from 1 to 4 carbon atoms, or an (imidazol-4-yl)methyl group optionally substituted at one of the nitrogen atoms by a straight-chain or branched alkyl radical having from 1 to 4 carbon atoms or by a benzyloxycarbonyl, 2,4-dinitrophenyl, fluorenomethoxycarbonyl, tosyl or benzyl radical,

$R_2$ and $R_3$, which may be the same or different, each represents a hydrogen atom or an alkyl radical having from 1 to 4 carbon atoms, or, in conjunction with the nitrogen atom to which they are attached, together form a 1-pyrrolidinyl, 1-piperidinyl, 4-morpholinyl or 4-($C_{1-4}$-alkyl)-1-piperazinyl radical,
the enantiomers and diastereoisomers thereof,
and also, when $R_1$ represents an (imidazol-4-yl)-methyl group optionally substituted at one of the nitrogen atoms by a straight-chain or branched alkyl radical having from 1 to 4 carbon atoms, or when $R_2$ and $R_3$ represent an N-alkyl-1-piperazinyl radical, the addition salts thereof with a pharmaceutically acceptable acid.

2. Compounds of the general formula I in which B together with the nitrogen and carbon atom to which it is attached represents a perhydroindole, perhydroisoquinoline or 2-azabicyclo[2,2,2]octane structure, the enantiomers and diastereoisomers thereof, and also, when $R_1$ represents an optionally substituted (imidazol-4-yl)methyl group, or when $R_2$ and $R_3$ represent an N-alkyl-1-piperazinyl radical, the addition salts thereof with a pharmaceutically acceptable acid.

3. Compounds of the general formula I in which B together with the nitrogen and carbon atom to which it is attached represents a perhydroindole structure, the enantiomers and diastereoisomers thereof, and also, when $R_1$ represents an optionally substituted (imidazol-4-yl)methyl group, or when $R_2$ and $R_3$ represent an N-alkyl-1-piperazinyl radical, the addition salts thereof with a pharmaceutically acceptable acid.

4. Compounds of the general formula I in which B together with the nitrogen and carbon atom to which it is attached represents a 2-azabicyclo[2,2,2]octane radical, the enantiomers and diastereoisomers thereof, and also, when $R_1$ represents an optionally substituted (imidazol-4-yl)methyl group, or when $R_2$ and $R_3$ represents an N-alkyl-1-piperazinyl radical, the addition salts thereof with a pharmaceutically acceptable acid.

5. PyroGlu-His-PHI-$NH_2$, the enantiomers of diastereoisomers thereof, as well as the addition salts thereof with a pharmaceutically acceptable acid, PHI representing the 2-carbonylperhydroindole radical.

6. Blc-His-PHI-$NH_2$, the enantiomers or diastereoisomers thereof, as well as the addition salts thereof with a pharmaceutically acceptable acid, PHI representing the 2-carbonylperhydroindole radical.

7. PyroGlu-Nva-PHI-$NH_2$, the enantiomers or diastereoisomers thereof, PHI representing the 2-carbonylperhydroindole radical.

8. PyroGlu-His-ABO-$NH_2$, the enantiomers or diastereoisomers thereof, as well as the addition salts thereof with a pharmaceutically acceptable acid, ABO representing a 3-carbonyl-2-azabicyclo[2,2,2]octane radical.

9. Homo-PyroGlu-His-ABO-$NH_2$, the enantiomers or diastereoisomers thereof, as well as the addition salts thereof with a pharmaceutically acceptable acid, ABO representing a 3-carbonyl-2-azabicyclo[2,2,2]octane radical.

10. Blc-His-ABO-$NH_2$, the enantiomers or diastereoisomers thereof, as well as the addition salts thereof with a pharmaceutically acceptable acid, ABO representing a 3-carbonyl-2-azabicyclo[2,2,2]octane radical.

11. Process for the preparation of the derivatives of formula I, characterised in that the amine function of an amino acid of formula II

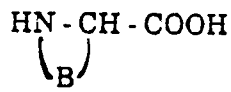

(II)

in which B together with the nitrogen and carbon atoms to which it is attached has the same meaning as in formula I, is protected by a tert.-butoxycarbonyl (tBoc) radical by the action of di-tert.-butyl carbonate, to form a derivative of formula III

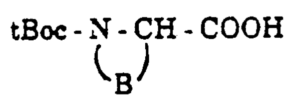

(III)

in which B has the same meaning as in formula I,
which is then converted, at a temperature of between 0 and −15°C, by the successive action of ethyl chloroformate and an amine of formula IV

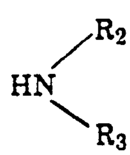

(IV)

in which $R_2$ and $R_3$ have the same meanings as in formula I,
into an amide of formula V:

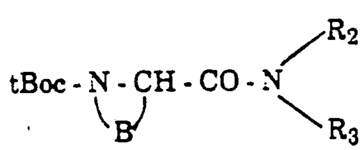

(V)

in which B, $R_2$ and $R_3$ have the same meanings as in formula I, which is de-protected by the action of gaseous hydrochloric acid in ethyl acetate, or of trifluoroacetic acid, to form a derivative of formula VI

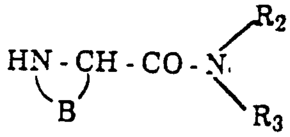

(VI)

in which B, $R_2$ and $R_3$ have the same meanings as in formula I, which is coupled with a protected amino acid of formula VII

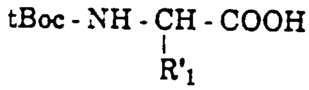

(VII)

in which $R'_1$ represents a hydrogen atom, a straight-chain or branched alkyl radical having from 1 to 4 carbon atoms, an (N-alkyl $(C_{1-4})$imidazol-4-yl)methyl radical, or an (imidazol-4-yl)methyl radical temporarily protected at one of the nitrogen atoms by a grouping such as a 2,4-dinitrophenyl, benzyloxycarbonyl, fluorenomethoxycarbonyl, tosyl or benzyl radical, to form a derivative of formula VIII

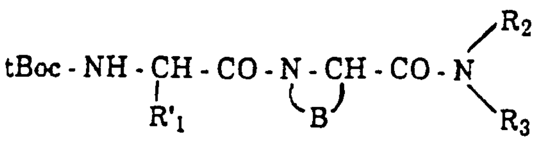

(VIII)

in which $R'_1$, $R_2$, $R_3$ and B have the same meanings as specified hereinbefore, which is de-protected as hereinbefore described to form a derivative of formula IX

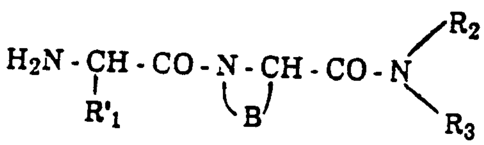

(IX)

in which $R'_1$, $R_2$, $R_3$ and B have the same meanings as hereinbefore, which is condensed with a derivative of formula X,

A—COOH (X)

in which A has the same meanings as in formula I

*either to form a derivative of formula I′

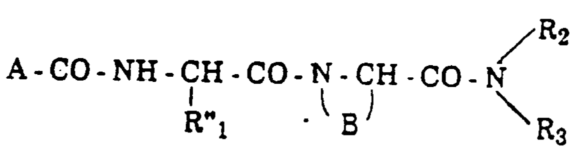

(I′)

in which A, B, $R_2$ and $R_3$ have the same meanings as hereinbefore and $R''_1$ represents a hydrogen atom, a straight-chain or branched alkyl radical having from 1 to 4 carbon atom atoms or an (N-alkyl($C_{1-4}$)imidazol-4-yl)methyl radical,
which derivative is, if desired, directly converted into its addition salt with a pharmaceutically acceptable acid when $R''_1$ represents an (N-alkylimidazolyl)methyl radical or when $R_2$ and $R_3$ represent, together with the nitrogen to which they are attached, a 1-piperazinyl radical substituted by an alkyl chain having from 1 to 4 carbon atoms, or is separated first into its enantiomers or diastereoisomers, then converted, if desired, when $R''_1$, $R_2$ and $R_3$ have the same meanings as those given hereinbefore, into its addition salt with a pharmaceutically acceptable acid,

*or to form a derivative of formula I''

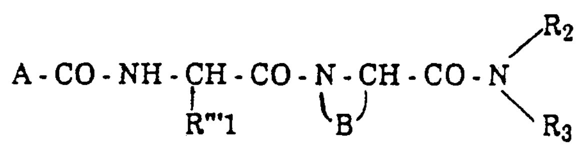

(I")

in which A, B, $R_2$ and $R_3$ have the same meanings as hereinbefore, and $R'''_1$ represents an (imidazol-4-yl)methyl group protected at one of the nitrogen atoms by a 2,4-dinitrophenyl, benzyloxycarbonyl, fluorenomethoxycarbonyl, tosyl or benzyl grouping, which derivative is de-protected to form a derivative of formula I'''

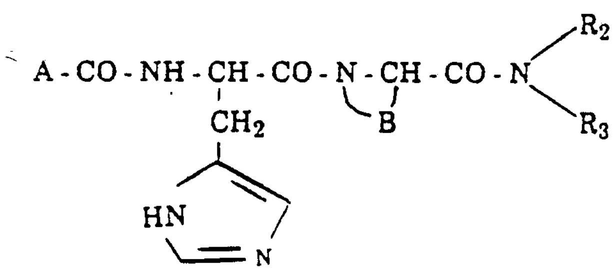

(I''')

in which A, B, $R_2$ and $R_3$ have the meanings given hereinbefore,
which is, if desired, converted into its addition salt with a pharmaceutically acceptable acid, or separated into its isomers then, if necessary, formed into a salt by a pharmaceutically acceptable acid.

12. Pharmaceutical composition containing as active ingredient at least one compound according to any one of claims 1 to 10, on its own or together with one or more pharmaceutically acceptable inert, nontoxic excipients or vehicles.

13. Pharmaceutical composition according to claim 12 for use in the treatment of disorders of senescence associated with normal or pathological ageing and disorders of the central nervous system.

**Claim for the Contracting State: AT**

Process for the preparation of compounds of the general formula I

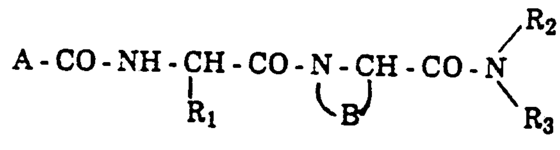

(I)

in which

A represents a 2-oxo-5-tetrahydrofuryl, 2-oxo-5-pyrrolidinyl or 2-oxo-6-piperidinyl grouping optionally substituted at the nitrogen atom by a straight-chain or branched alkyl grouping having from 1 to 4 carbon atoms, or represents a 2,6-dioxo-1,2,3,6-tetrahydro-4-pyrimidinyl grouping,

B represents, together with the carbon and nitrogen atom to which it is attached, a saturated polycyclic structure selected from the group comprising perhydroindole, perhydroisoindole, perhydroquinoline, perhydroisoquinoline, cyclopenta[b]pyrrole and 2-azabicyclo[2,2,2]octane,

$R_1$ represents a hydrogen atom, a straight-chain or branched alkyl radical having from 1 to 4 carbon atoms, or an (imidazol-4-yl)methyl group optionally substituted at one of the nitrogen atoms by a straight-chain or branched alkyl radical having from 1 to 4 carbon atoms or by a benzyloxycarbonyl, 2,4-dinitrophenyl, fluorenomethoxycarbonyl, tosyl or benzyl radical,

$R_2$ and $R_3$, which may be the same or different, each represents a hydrogen atom or an alkyl radical having from 1 to 4 carbon atoms, or, in conjunction with the nitrogen atom to which they are attached, together form a 1-pyrrolidinyl, 1-piperidinyl, 4-morpholinyl or 4-($C_{1-4}$-alkyl)-1-piperazinyl radical,

the enantiomers and diastereoisomers thereof,

and also, when $R_1$ represents an (imidazol-4-yl)-methyl group optionally substituted at one of the nitrogen atoms by a straight-chain or branched alkyl radical having from 1 to 4 carbon atoms, or when $R_2$ and $R_3$ represent an N-alkyl-1-piperazinyl radical, the addition salts thereof with a pharmaceutically acceptable acid, characterised in that the amine function of an amino acid of formula II

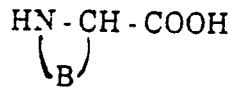

(II)

in which B together with the nitrogen and carbon atoms to which it is attached has the same meaning as in formula I, is protected by a tert.-butoxycarbonyl (tBoc) radical by the action of di-tert.-butyl carbonate, to form a derivative of formula III

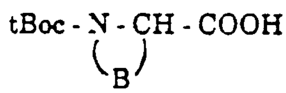

(III)

in which B has the same meaning as in formula I,

which is then converted, at a temperature of between 0 and −15°C, by the successive action of ethyl chloroformate and an amine of formula IV

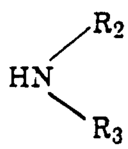

(IV)

in which $R_2$ and $R_3$ have the same meanings as in formula I,

into an amide of formula V:

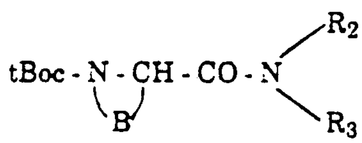

(V)

in which B, $R_2$ and $R_3$ have the same meanings as in formula I,

which is de-protected by the action of gaseous hydrochloric acid in ethyl acetate, or of trifluoroacetic acid, to form a derivative of formula VI

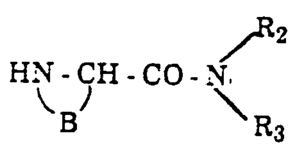

(VI)

in which B, $R_2$ and $R_3$ have the same meanings as in formula I, which is coupled with a protected amino acid of formula VII

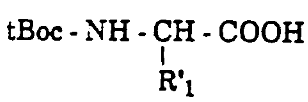

(VII)

in which $R'_1$ represents a hydrogen atom, a straight-chain or branched alkyl radical having from 1 to 4 carbon atoms, an (N-alkyl ($C_{1-4}$)imidazol-4-yl)methyl radical, or an (imidazol-4-yl)methyl radical temporarily protected at one of the nitrogen atoms by a grouping such as a 2,4-dinitrophenyl, benzyloxycarbonyl, fluorenomethoxycarbonyl, tosyl or benzyl radical,

to form a derivative of formula VIII

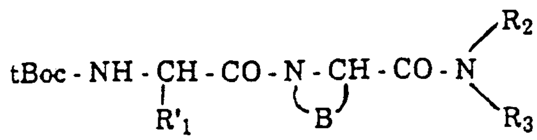

(VIII)

in which $R'_1$, $R_2$, $R_3$ and B have the same meanings as specified hereinbefore, which is de-protected as hereinbefore described to form a derivative of formula IX

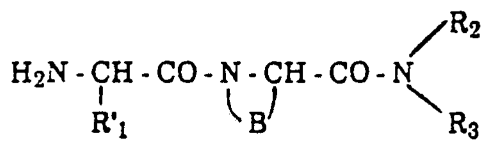

(IX)

in which $R'_1$, $R_2$, $R_3$ and B have the same meanings as hereinbefore, which is condensed with a derivative of formula X,

A—COOH (X)

in which A has the same meanings as in formula I

*either to form a derivative of formula I′

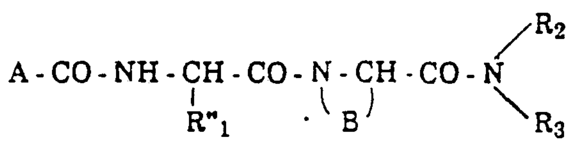

(I′)

in which A, B, $R_2$ and $R_3$ have the same meanings as hereinbefore and $R''_1$ represents a hydrogen atom, a straight-chain or branched alkyl radical having from 1 to 4 carbon atoms or an (N-alkyl($C_{1-4}$)imidazol-4-yl)methyl radical,

which derivative is, if desired, directly converted into its addition salt with a pharmaceutically acceptable acid when $R''_1$ represents an (N-alkylimidazolyl)methyl radical or when $R_2$ and $R_3$ represent, together with the nitrogen to which they are attached, a 1-piperazinyl radical substituted by an alkyl chain having from 1 to 4 carbon atoms, or is separated first into its enantiomers or diastereoisomers, then converted, if desired, when $R''_1$, $R_2$ and $R_3$ have the same meanings as those given hereinbefore, into its addition salt with a pharmaceutically acceptable acid,

*or to form a derivative of formula I″

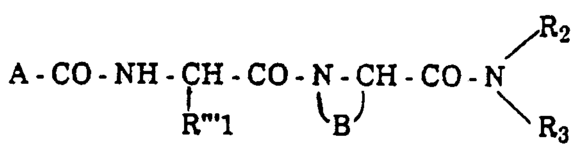

(I″)

in which A, B, $R_2$ and $R_3$ have the same meanings as hereinbefore, and $R'''_1$ represents an (imidazol-4-yl)methyl group protected at one of the nitrogen atoms by a 2,4-dinitrophenyl, benzyloxycarbonyl, fluorenomethoxycarbonyl, tosyl or benzyl grouping, which derivative is de-protected to form a derivative of formula I‴

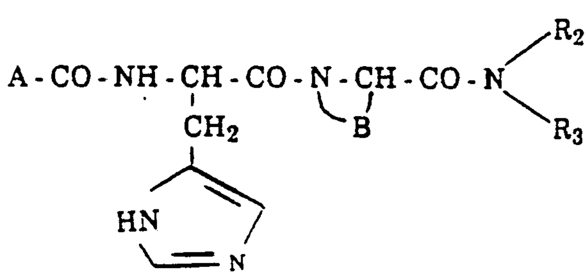

(I‴)

in which A, B, $R_2$ and $R_3$ have the meanings given hereinbefore,

which is, if desired, converted into its addition salt with a pharmaceutically acceptable acid, or separated into its isomers then, if necessary, formed into a salt by a pharmaceutically acceptable acid.